# EUROPEAN PATENT APPLICATION

(11) **EP 2 476 703 A1**
(43) Date of publication of application: **18.07.2012**
(21) Application number: 11150941.0
(22) Date of filing: 14.01.2011
(51) Int. Cl.: C07K 16/12, C07K 16/18

(54) **Human antibodies cross-reacting with a bacterial and a self antigen from atherosclerotic plaques**

(71) Applicant: Bracco Imaging S.p.A, 20134 Milano (IT)
(72) Inventor: Burioni, Roberto, 20132 Milano (IT); Clementi, Massimo, 20132 Milano (IT); Canducci, Filippo, 20132 Milano (IT)
(74) Representative: Merli, Silvia

(57) **Abstract**

The present invention refers to human antibodies able to immunologically recognize both human transgelin or fragments thereof and a protein with at least 50% similarity to OmpK36 (Outer membrane protein, Klebsiella, K36; GI: 295881594) or fragments thereof. Human transgelin is preferably transgelin 1 (Accession N° Q01995, GI:48255907).

The antibodies further recognize an antigen in the atherosclerotic plaque and are useful for the preparation of immunodiagnostic reagents or assays to detect atherogenic diseases.

SEQUENCE LISTING

<110>  Bracco Imaging SpA
       ?, ?

<120>  HUMAN ANTIBODIES CROSS-REACTING WITH A BACTERIAL AND A SELF
       ANTIGEN FROM ATHEROSCLEROTIC PLAQUES

<130>  ?

<160>  79

<170>  PatentIn version 3.3

<210>  1
<211>  445
<212>  DNA
<213>  Homo sapiens


<220>
<221>  CDS
<222>  (1)..(444)
<223>  Fab7816HC

<400>  1
ctc gag tcg ggc cca gga cag gtg aag cct tca cag acc ctg tcc ctc       48
Leu Glu Ser Gly Pro Gly Gln Val Lys Pro Ser Gln Thr Leu Ser Leu
1               5                   10                  15

acc tgc act gtc tct ggt ggc tcc atc ggc agt ggt tct tac tcc tgg       96
Thr Cys Thr Val Ser Gly Gly Ser Ile Gly Ser Gly Ser Tyr Ser Trp
            20                  25                  30

aac tgg atc cgg cag ccc gcc ggg agg gga ctg gag tgg att ggg cga      144
Asn Trp Ile Arg Gln Pro Ala Gly Arg Gly Leu Glu Trp Ile Gly Arg
        35                  40                  45

atc tct gac agt ggg aac acc aat ttc aac ccc tcc ctc aag agt cga      192
Ile Ser Asp Ser Gly Asn Thr Asn Phe Asn Pro Ser Leu Lys Ser Arg
    50                  55                  60

gtc acc atg tca gtg gac acg tcc aag aac cag ttc gcc ctg aaa ctg      240
Val Thr Met Ser Val Asp Thr Ser Lys Asn Gln Phe Ala Leu Lys Leu
65                  70                  75                  80

acc tct gtg acc gcc gca gac acg gcc aca tat ttc tgt gcg aga ggg      288
Thr Ser Val Thr Ala Ala Asp Thr Ala Thr Tyr Phe Cys Ala Arg Gly
                85                  90                  95

aga ggt att ttg act ggt ctc ttt gac tat tgg ggc cag gga tcc ctg      336
Arg Gly Ile Leu Thr Gly Leu Phe Asp Tyr Trp Gly Gln Gly Ser Leu
            100                 105                 110

gtc tcc gtc tcc tca gcc tcc acc aag ggc cca tcg gtc ttc ccc ctg      384
Val Ser Val Ser Ser Ala Ser Thr Lys Gly Pro Ser Val Phe Pro Leu
        115                 120                 125

gca ccc tcc tcc aag agc acc tct ggg ggc aca gcg gcc ctg ggc tgc      432
Ala Pro Ser Ser Lys Ser Thr Ser Gly Gly Thr Ala Ala Leu Gly Cys
    130                 135                 140

ctg gtc aag gac t                                                    445
Leu Val Lys Asp
145


<210>  2
<211>  148
<212>  PRT

<213>  Homo sapiens

<400>  2

Leu Glu Ser Gly Pro Gly Gln Val Lys Pro Ser Gln Thr Leu Ser Leu
1               5                   10                  15

Thr Cys Thr Val Ser Gly Gly Ser Ile Gly Ser Gly Ser Tyr Ser Trp
            20                  25                  30

Asn Trp Ile Arg Gln Pro Ala Gly Arg Gly Leu Glu Trp Ile Gly Arg
        35                  40                  45

Ile Ser Asp Ser Gly Asn Thr Asn Phe Asn Pro Ser Leu Lys Ser Arg
    50                  55                  60

Val Thr Met Ser Val Asp Thr Ser Lys Asn Gln Phe Ala Leu Lys Leu
65                  70                  75                  80

Thr Ser Val Thr Ala Ala Asp Thr Ala Thr Tyr Phe Cys Ala Arg Gly
                85                  90                  95

Arg Gly Ile Leu Thr Gly Leu Phe Asp Tyr Trp Gly Gln Gly Ser Leu
            100                 105                 110

Val Ser Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp
145


<210>  3
<211>  316
<212>  DNA
<213>  Homo sapiens


<220>
<221>  CDS
<222>  (1)..(315)
<223>  Fab7816LC

<400>  3
tct cca gac tcc ctg gct gtg tct ccg ggc ggg agg gcc acc atc aag       48
Ser Pro Asp Ser Leu Ala Val Ser Pro Gly Gly Arg Ala Thr Ile Lys
1               5                   10                  15

tgc gcg tcc agc cag agt gtt ttg gac aac tcc aac cat aag aac tcc       96
Cys Ala Ser Ser Gln Ser Val Leu Asp Asn Ser Asn His Lys Asn Ser
            20                  25                  30

ttg gcg tgg tac cag cag aaa cca gga ctg cct cct aaa ctg ctc att      144
Leu Ala Trp Tyr Gln Gln Lys Pro Gly Leu Pro Pro Lys Leu Leu Ile
        35                  40                  45

tac tgg gca tct acc cgg tat tcc ggg gtc cct gac cga ttc agt ggc      192
Tyr Trp Ala Ser Thr Arg Tyr Ser Gly Val Pro Asp Arg Phe Ser Gly

50                  55                  60

agt ggg tct ggg aca gat ttc act ctc acc atc aac aac ctg cag gct        240
Ser Gly Ser Gly Thr Asp Phe Thr Leu Thr Ile Asn Asn Leu Gln Ala
65                  70                  75                  80

gcc gat gtg gca gtt tat ttc tgt cag caa tat tat agt act ccg tgg        288
Ala Asp Val Ala Val Tyr Phe Cys Gln Gln Tyr Tyr Ser Thr Pro Trp
                85                  90                  95

acc ttc ggc cag ggg acc aag gtg gag c                                  316
Thr Phe Gly Gln Gly Thr Lys Val Glu
            100                 105


<210>  4
<211>  105
<212>  PRT
<213>  Homo sapiens

<400>  4

Ser Pro Asp Ser Leu Ala Val Ser Pro Gly Gly Arg Ala Thr Ile Lys
1               5                   10                  15


Cys Ala Ser Ser Gln Ser Val Leu Asp Asn Ser Asn His Lys Asn Ser
            20                  25                  30


Leu Ala Trp Tyr Gln Gln Lys Pro Gly Leu Pro Pro Lys Leu Leu Ile
        35                  40                  45


Tyr Trp Ala Ser Thr Arg Tyr Ser Gly Val Pro Asp Arg Phe Ser Gly
    50                  55                  60


Ser Gly Ser Gly Thr Asp Phe Thr Leu Thr Ile Asn Asn Leu Gln Ala
65                  70                  75                  80


Ala Asp Val Ala Val Tyr Phe Cys Gln Gln Tyr Tyr Ser Thr Pro Trp
                85                  90                  95


Thr Phe Gly Gln Gly Thr Lys Val Glu
            100                 105


<210>  5
<211>  455
<212>  DNA
<213>  Homo sapiens


<220>
<221>  CDS
<222>  (1)..(444)
<223>  Fab5LcxHC

<400>  5
ggc cca gga ctg gtg aag cct tcg ggg acc ctg tcc ctc acc tgc gct         48
Gly Pro Gly Leu Val Lys Pro Ser Gly Thr Leu Ser Leu Thr Cys Ala
1               5                   10                  15

gtc tct ggt ggc tcc atc agc agt agt aac tgg tgg att tgg gtc cgc         96
Val Ser Gly Gly Ser Ile Ser Ser Ser Asn Trp Trp Ile Trp Val Arg
            20                  25                  30

cag ccc cca ggg aag agg ctg gag tgg att gga gaa atc gat cat agt          144
Gln Pro Pro Gly Lys Arg Leu Glu Trp Ile Gly Glu Ile Asp His Ser
        35                  40                  45

ggg act acc aac tac aac ccg tcc ctc aag agt cga gtc acc atg tca          192
Gly Thr Thr Asn Tyr Asn Pro Ser Leu Lys Ser Arg Val Thr Met Ser
    50                  55                  60

gtg gtc aag tcc aag aac cag ttc tcc ctg aag ctg agc tct gtg acc          240
Val Val Lys Ser Lys Asn Gln Phe Ser Leu Lys Leu Ser Ser Val Thr
65                  70                  75                  80

gcc gcg gac acg gcc gtc tat tac tgt gcg aga gga gca aag gat aac          288
Ala Ala Asp Thr Ala Val Tyr Tyr Cys Ala Arg Gly Ala Lys Asp Asn
                85                  90                  95

tgg gga ttc gac tac tgg ggc cag gga atc ttg gtc acc gtc tcc tca          336
Trp Gly Phe Asp Tyr Trp Gly Gln Gly Ile Leu Val Thr Val Ser Ser
            100                 105                 110

gcc tcc acc aag ggc cca tcg gtc ttc ccc ctg gca ccc tcc tcc aag          384
Ala Ser Thr Lys Gly Pro Ser Val Phe Pro Leu Ala Pro Ser Ser Lys
        115                 120                 125

agc acc tct ggg ggc aca gcg gcc ctg ggc tgc ctg gtc aag gac tac          432
Ser Thr Ser Gly Gly Thr Ala Ala Leu Gly Cys Leu Val Lys Asp Tyr
    130                 135                 140

ttc ccc gaa ccg gtgacggtgt c                                             455
Phe Pro Glu Pro
145


<210>  6
<211>  148
<212>  PRT
<213>  Homo sapiens

<400>  6

Gly Pro Gly Leu Val Lys Pro Ser Gly Thr Leu Ser Leu Thr Cys Ala
1               5                   10                  15


Val Ser Gly Gly Ser Ile Ser Ser Ser Asn Trp Trp Ile Trp Val Arg
            20                  25                  30


Gln Pro Pro Gly Lys Arg Leu Glu Trp Ile Gly Glu Ile Asp His Ser
        35                  40                  45


Gly Thr Thr Asn Tyr Asn Pro Ser Leu Lys Ser Arg Val Thr Met Ser
    50                  55                  60


Val Val Lys Ser Lys Asn Gln Phe Ser Leu Lys Leu Ser Ser Val Thr
65                  70                  75                  80


Ala Ala Asp Thr Ala Val Tyr Tyr Cys Ala Arg Gly Ala Lys Asp Asn
                85                  90                  95


Trp Gly Phe Asp Tyr Trp Gly Gln Gly Ile Leu Val Thr Val Ser Ser
            100                 105                 110


Ala Ser Thr Lys Gly Pro Ser Val Phe Pro Leu Ala Pro Ser Ser Lys
        115                 120                 125

Ser Thr Ser Gly Gly Thr Ala Ala Leu Gly Cys Leu Val Lys Asp Tyr
    130                 135                 140

Phe Pro Glu Pro
145

<210>  7
<211>  376
<212>  DNA
<213>  Homo sapiens

<220>
<221>  CDS
<222>  (1)..(375)
<223>  Fab5LcxLC

<400>  7
gcc atg gcc gag ctc acc cag tct cca tcc tcc ctg tct gca tct gta        48
Ala Met Ala Glu Leu Thr Gln Ser Pro Ser Ser Leu Ser Ala Ser Val
1               5                   10                  15

gga gac aga gtc gcc atc act tgc cgg gca agt cag acc atc ctc gac        96
Gly Asp Arg Val Ala Ile Thr Cys Arg Ala Ser Gln Thr Ile Leu Asp
            20                  25                  30

tat tta aat tgg tat cag cag cga ccc ggg aaa gcc cct aag ctc ctc       144
Tyr Leu Asn Trp Tyr Gln Gln Arg Pro Gly Lys Ala Pro Lys Leu Leu
        35                  40                  45

atc tat tct gca acc cgt tta caa act ggg gtc cca tca agg ttc act       192
Ile Tyr Ser Ala Thr Arg Leu Gln Thr Gly Val Pro Ser Arg Phe Thr
    50                  55                  60

ggc agt aga tct ggg aca gat ttc act ctc acc atc agc agc cta caa       240
Gly Ser Arg Ser Gly Thr Asp Phe Thr Leu Thr Ile Ser Ser Leu Gln
65                  70                  75                  80

cct gaa gaa ttt gca act tat tac tgt caa cac gat tac aat gac cct       288
Pro Glu Glu Phe Ala Thr Tyr Tyr Cys Gln His Asp Tyr Asn Asp Pro
                85                  90                  95

cga act ttc ggc cag ggg acc aag gtg gaa gtc aaa cga act gtg gct       336
Arg Thr Phe Gly Gln Gly Thr Lys Val Glu Val Lys Arg Thr Val Ala
            100                 105                 110

gca cca tct gtc ttc atc ttc ccg cca tct gat gag cag t                 376
Ala Pro Ser Val Phe Ile Phe Pro Pro Ser Asp Glu Gln
        115                 120                 125

<210>  8
<211>  125
<212>  PRT
<213>  Homo sapiens

<400>  8

Ala Met Ala Glu Leu Thr Gln Ser Pro Ser Ser Leu Ser Ala Ser Val
1               5                   10                  15

Gly Asp Arg Val Ala Ile Thr Cys Arg Ala Ser Gln Thr Ile Leu Asp
            20                  25                  30

Tyr Leu Asn Trp Tyr Gln Gln Arg Pro Gly Lys Ala Pro Lys Leu Leu
        35                  40                  45

Ile Tyr Ser Ala Thr Arg Leu Gln Thr Gly Val Pro Ser Arg Phe Thr
    50                  55                  60

Gly Ser Arg Ser Gly Thr Asp Phe Thr Leu Thr Ile Ser Ser Leu Gln
65                  70                  75                  80

Pro Glu Glu Phe Ala Thr Tyr Tyr Cys Gln His Asp Tyr Asn Asp Pro
                85                  90                  95

Arg Thr Phe Gly Gln Gly Thr Lys Val Glu Val Lys Arg Thr Val Ala
            100                 105                 110

Ala Pro Ser Val Phe Ile Phe Pro Pro Ser Asp Glu Gln
        115                 120                 125

<210>  9
<211>  476
<212>  DNA
<213>  Homo sapiens

<220>
<221>  CDS
<222>  (1)..(474)
<223>  Fab1630HC

<400>  9
gga gga ggc ttg gtc aag cct ggc ggg tcc ctg aga ctc tcc tgc aca      48
Gly Gly Gly Leu Val Lys Pro Gly Gly Ser Leu Arg Leu Ser Cys Thr
1               5                   10                  15

gcc tct gga ttc act ttc agt aac ggc tgg atg agc tgg gtc cgc cag      96
Ala Ser Gly Phe Thr Phe Ser Asn Gly Trp Met Ser Trp Val Arg Gln
            20                  25                  30

gct cct ggg aag ggg ctg gag tgg gtc ggc cgg att aga agc aac ccc      144
Ala Pro Gly Lys Gly Leu Glu Trp Val Gly Arg Ile Arg Ser Asn Pro
        35                  40                  45

gac ggt ggc aca aca gac tac gct gca ccc ttc aaa ggc aga ttc acc      192
Asp Gly Gly Thr Thr Asp Tyr Ala Ala Pro Phe Lys Gly Arg Phe Thr
    50                  55                  60

atc tca aga gat gat tca aaa aat aca ttg ttt ctg caa gtg acc agc      240
Ile Ser Arg Asp Asp Ser Lys Asn Thr Leu Phe Leu Gln Val Thr Ser
65                  70                  75                  80

ctg aaa acc gag gac aca ggc gtc tat tac tgc atc aca gat cgg ggt      288
Leu Lys Thr Glu Asp Thr Gly Val Tyr Tyr Cys Ile Thr Asp Arg Gly
                85                  90                  95

gac tgg aag tgg ggg gtc cct agg gac ctc acc tac tgg ggc cag gga      336
Asp Trp Lys Trp Gly Val Pro Arg Asp Leu Thr Tyr Trp Gly Gln Gly
            100                 105                 110

acc ctg gtc acc gtc tcc tca gcc tcc acc aag ggc cca tcg gtc ttc      384
Thr Leu Val Thr Val Ser Ser Ala Ser Thr Lys Gly Pro Ser Val Phe
        115                 120                 125

ccc ctg gca ccc tcc tcc aag agc acc tct ggg ggc aca gcg gcc ctg      432
Pro Leu Ala Pro Ser Ser Lys Ser Thr Ser Gly Gly Thr Ala Ala Leu

130                 135                 140

ggc tgc ctg gtc aag gac tac ttc ccc gaa ccg gtg acg gtg tc          476
Gly Cys Leu Val Lys Asp Tyr Phe Pro Glu Pro Val Thr Val
145                 150                 155

<210>  10
<211>  158
<212>  PRT
<213>  Homo sapiens

<400>  10

Gly Gly Gly Leu Val Lys Pro Gly Gly Ser Leu Arg Leu Ser Cys Thr
1               5                   10                  15

Ala Ser Gly Phe Thr Phe Ser Asn Gly Trp Met Ser Trp Val Arg Gln
            20                  25                  30

Ala Pro Gly Lys Gly Leu Glu Trp Val Gly Arg Ile Arg Ser Asn Pro
        35                  40                  45

Asp Gly Gly Thr Thr Asp Tyr Ala Ala Pro Phe Lys Gly Arg Phe Thr
    50                  55                  60

Ile Ser Arg Asp Asp Ser Lys Asn Thr Leu Phe Leu Gln Val Thr Ser
65                  70                  75                  80

Leu Lys Thr Glu Asp Thr Gly Val Tyr Tyr Cys Ile Thr Asp Arg Gly
                85                  90                  95

Asp Trp Lys Trp Gly Val Pro Arg Asp Leu Thr Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val
145                 150                 155

<210>  11
<211>  374
<212>  DNA
<213>  Homo sapiens

<220>
<221>  CDS
<222>  (1)..(372)
<223>  Fab1630LC

<400>  11
gcc atg gcc gag ctc acg cag tct cca gac acc ctg tct ttg tct cca          48
Ala Met Ala Glu Leu Thr Gln Ser Pro Asp Thr Leu Ser Leu Ser Pro
1               5                   10                  15

ggg gaa aga gcc acc ctc tcc tgt agg gcc agt cag agt gtc gac agc      96
Gly Glu Arg Ala Thr Leu Ser Cys Arg Ala Ser Gln Ser Val Asp Ser
            20                  25                  30

aac tac tta gcc tgg ttc cag cag aag cct ggc cag gct ccc agg ctc      144
Asn Tyr Leu Ala Trp Phe Gln Gln Lys Pro Gly Gln Ala Pro Arg Leu
        35                  40                  45

ctc att tat ggt gcg tat agc agg gcc act ggc atc cca gac agg ttc      192
Leu Ile Tyr Gly Ala Tyr Ser Arg Ala Thr Gly Ile Pro Asp Arg Phe
    50                  55                  60

agt ggc agt ggg tct ggg aca gac ttc act ctc acc atc agc aga ctg      240
Ser Gly Ser Gly Ser Gly Thr Asp Phe Thr Leu Thr Ile Ser Arg Leu
65                  70                  75                  80

gag cct gag gat ttt gtc gtg tat tac tgt cag cag tat ctt agc ccg      288
Glu Pro Glu Asp Phe Val Val Tyr Tyr Cys Gln Gln Tyr Leu Ser Pro
                85                  90                  95

ccg atc acc ttc ggc caa ggg aca cga ctg gag act aaa cga act gtg      336
Pro Ile Thr Phe Gly Gln Gly Thr Arg Leu Glu Thr Lys Arg Thr Val
            100                 105                 110

gct gca cca tct gtc ttc atc ttc ccg cca tct gat ga                   374
Ala Ala Pro Ser Val Phe Ile Phe Pro Pro Ser Asp
        115                 120


<210>  12
<211>  124
<212>  PRT
<213>  Homo sapiens

<400>  12

Ala Met Ala Glu Leu Thr Gln Ser Pro Asp Thr Leu Ser Leu Ser Pro
1               5                   10                  15


Gly Glu Arg Ala Thr Leu Ser Cys Arg Ala Ser Gln Ser Val Asp Ser
            20                  25                  30


Asn Tyr Leu Ala Trp Phe Gln Gln Lys Pro Gly Gln Ala Pro Arg Leu
        35                  40                  45


Leu Ile Tyr Gly Ala Tyr Ser Arg Ala Thr Gly Ile Pro Asp Arg Phe
    50                  55                  60


Ser Gly Ser Gly Ser Gly Thr Asp Phe Thr Leu Thr Ile Ser Arg Leu
65                  70                  75                  80


Glu Pro Glu Asp Phe Val Val Tyr Tyr Cys Gln Gln Tyr Leu Ser Pro
                85                  90                  95


Pro Ile Thr Phe Gly Gln Gly Thr Arg Leu Glu Thr Lys Arg Thr Val
            100                 105                 110


Ala Ala Pro Ser Val Phe Ile Phe Pro Pro Ser Asp
        115                 120


<210>  13
<211>  306

<212>  DNA
<213>  Homo sapiens

<220>
<221>  CDS
<222>  (1)..(306)
<223>  Fab248HC

<400>  13
ctc gag tct ggg gga ggc ttg gtc aag cct gga ggg tcc ctg agg ctc      48
Leu Glu Ser Gly Gly Gly Leu Val Lys Pro Gly Gly Ser Leu Arg Leu
1               5                   10                  15

tcc tgt gca gcc tct gga ttc acc ttc agt gac tac tac atg agt tgg      96
Ser Cys Ala Ala Ser Gly Phe Thr Phe Ser Asp Tyr Tyr Met Ser Trp
            20                  25                  30

atc cgc cag gct cca ggg aag ggg ctg gaa ttt ata tca tac att agt      144
Ile Arg Gln Ala Pro Gly Lys Gly Leu Glu Phe Ile Ser Tyr Ile Ser
        35                  40                  45

agt ggt ggt gac acc ata cac cac gca gac tct gtg aag ggc cga ttc      192
Ser Gly Gly Asp Thr Ile His His Ala Asp Ser Val Lys Gly Arg Phe
    50                  55                  60

acc atc tcc agg gac aac gcc aag aag tca ctg tat ctc caa atg aac      240
Thr Ile Ser Arg Asp Asn Ala Lys Lys Ser Leu Tyr Leu Gln Met Asn
65                  70                  75                  80

agc ctg aga gtc gag gac acg gcc gta tat tac tgt gcg tgc cgt ggg      288
Ser Leu Arg Val Glu Asp Thr Ala Val Tyr Tyr Cys Ala Cys Arg Gly
                85                  90                  95

gtc tgg ggc cag gga acc                                              306
Val Trp Gly Gln Gly Thr
            100

<210>  14
<211>  102
<212>  PRT
<213>  Homo sapiens

<400>  14

Leu Glu Ser Gly Gly Gly Leu Val Lys Pro Gly Gly Ser Leu Arg Leu
1               5                   10                  15

Ser Cys Ala Ala Ser Gly Phe Thr Phe Ser Asp Tyr Tyr Met Ser Trp
            20                  25                  30

Ile Arg Gln Ala Pro Gly Lys Gly Leu Glu Phe Ile Ser Tyr Ile Ser
        35                  40                  45

Ser Gly Gly Asp Thr Ile His His Ala Asp Ser Val Lys Gly Arg Phe
    50                  55                  60

Thr Ile Ser Arg Asp Asn Ala Lys Lys Ser Leu Tyr Leu Gln Met Asn
65                  70                  75                  80

Ser Leu Arg Val Glu Asp Thr Ala Val Tyr Tyr Cys Ala Cys Arg Gly
                85                  90                  95

Val Trp Gly Gln Gly Thr
            100

<210>  15
<211>  291
<212>  DNA
<213>  Homo sapiens

<220>
<221>  CDS
<222>  (1)..(291)
<223>  Fab248LC

<400>  15
tct cca gcc acc gtg tct gtg tct cca ggg gaa aga gcc acc ctc tcc        48
Ser Pro Ala Thr Val Ser Val Ser Pro Gly Glu Arg Ala Thr Leu Ser
1               5                   10                  15

tgc agg gcc agt cag agt att agt ttc cac tta gcc tgg tac cag cag        96
Cys Arg Ala Ser Gln Ser Ile Ser Phe His Leu Ala Trp Tyr Gln Gln
            20                  25                  30

aaa cct ggc cag gct ccc agt ctc ctc atc tac gga aca tcc acc agg       144
Lys Pro Gly Gln Ala Pro Ser Leu Leu Ile Tyr Gly Thr Ser Thr Arg
        35                  40                  45

gcc act ggt atc cca gcc agg ttc agt ggc agt ggg tct ggg aca gag       192
Ala Thr Gly Ile Pro Ala Arg Phe Ser Gly Ser Gly Ser Gly Thr Glu
    50                  55                  60

ttc act ctc acc atc agc agc ctg cag tct gaa gat tct gcg gtt tat       240
Phe Thr Leu Thr Ile Ser Ser Leu Gln Ser Glu Asp Ser Ala Val Tyr
65                  70                  75                  80

tac tgt cag cag tat cat aac tgg cct ccc ctc act ttc ggc gga ggg       288
Tyr Cys Gln Gln Tyr His Asn Trp Pro Pro Leu Thr Phe Gly Gly Gly
                85                  90                  95

acc                                                                   291
Thr

<210>  16
<211>  97
<212>  PRT
<213>  Homo sapiens

<400>  16

Ser Pro Ala Thr Val Ser Val Ser Pro Gly Glu Arg Ala Thr Leu Ser
1               5                   10                  15

Cys Arg Ala Ser Gln Ser Ile Ser Phe His Leu Ala Trp Tyr Gln Gln
            20                  25                  30

Lys Pro Gly Gln Ala Pro Ser Leu Leu Ile Tyr Gly Thr Ser Thr Arg
        35                  40                  45

Ala Thr Gly Ile Pro Ala Arg Phe Ser Gly Ser Gly Ser Gly Thr Glu
    50                  55                  60

Phe Thr Leu Thr Ile Ser Ser Leu Gln Ser Glu Asp Ser Ala Val Tyr
65                  70                  75                  80

Tyr Cys Gln Gln Tyr His Asn Trp Pro Pro Leu Thr Phe Gly Gly Gly
                85                  90                  95

Thr

<210>  17
<211>  440
<212>  DNA
<213>  Homo sapiens

<220>
<221>  CDS
<222>  (1)..(438)
<223>  PL12HC22

<400>  17
ggg gct gag gtg aag aag act ggg tcc tca gtg aag gtg tcc tgc atg        48
Gly Ala Glu Val Lys Lys Thr Gly Ser Ser Val Lys Val Ser Cys Met
1               5                   10                  15

gtc tcc gga aac agc ttc acc cag cgt ttc ctg cac tgg gtg cga cag        96
Val Ser Gly Asn Ser Phe Thr Gln Arg Phe Leu His Trp Val Arg Gln
            20                  25                  30

gcc ccc gga caa gcg ctt gag tgg atg ggg tgg atc aca cct ttc agt       144
Ala Pro Gly Gln Ala Leu Glu Trp Met Gly Trp Ile Thr Pro Phe Ser
        35                  40                  45

gga aat acc tac tac gca cag aaa ttc cag gac aga ctc acc att acg       192
Gly Asn Thr Tyr Tyr Ala Gln Lys Phe Gln Asp Arg Leu Thr Ile Thr
    50                  55                  60

ggg gac agg tct gtg agt aca gcc tac atg gag ttg agc agc ctg aga       240
Gly Asp Arg Ser Val Ser Thr Ala Tyr Met Glu Leu Ser Ser Leu Arg
65                  70                  75                  80

tct gac gac aca gcc atc tat tac tgt gtg att ttt ggt ctt gac tac       288
Ser Asp Asp Thr Ala Ile Tyr Tyr Cys Val Ile Phe Gly Leu Asp Tyr
                85                  90                  95

tgg ggc aag gga acc ctg gtc acc gtc tcc tca gcc tcc acc aag ggc       336
Trp Gly Lys Gly Thr Leu Val Thr Val Ser Ser Ala Ser Thr Lys Gly
            100                 105                 110

cca tcg gtc ttc ccc ctg gca ccc tcc tcc aag agc acc tct ggg ggc       384
Pro Ser Val Phe Pro Leu Ala Pro Ser Ser Lys Ser Thr Ser Gly Gly
        115                 120                 125

aca gcg gcc ctg ggc tgc ctg gtc aag gac tac ttc ccc gaa ccg gtg       432
Thr Ala Ala Leu Gly Cys Leu Val Lys Asp Tyr Phe Pro Glu Pro Val
    130                 135                 140

acg gtg tc                                                            440
Thr Val
145

<210>  18
<211>  146
<212>  PRT
<213>  Homo sapiens

<400>  18

Gly Ala Glu Val Lys Lys Thr Gly Ser Ser Val Lys Val Ser Cys Met
1               5                   10                  15

Val Ser Gly Asn Ser Phe Thr Gln Arg Phe Leu His Trp Val Arg Gln
            20                  25                  30

Ala Pro Gly Gln Ala Leu Glu Trp Met Gly Trp Ile Thr Pro Phe Ser
        35                  40                  45

Gly Asn Thr Tyr Tyr Ala Gln Lys Phe Gln Asp Arg Leu Thr Ile Thr
    50                  55                  60

Gly Asp Arg Ser Val Ser Thr Ala Tyr Met Glu Leu Ser Ser Leu Arg
65                  70                  75                  80

Ser Asp Asp Thr Ala Ile Tyr Tyr Cys Val Ile Phe Gly Leu Asp Tyr
                85                  90                  95

Trp Gly Lys Gly Thr Leu Val Thr Val Ser Ser Ala Ser Thr Lys Gly
            100                 105                 110

Pro Ser Val Phe Pro Leu Ala Pro Ser Ser Lys Ser Thr Ser Gly Gly
        115                 120                 125

Thr Ala Ala Leu Gly Cys Leu Val Lys Asp Tyr Phe Pro Glu Pro Val
    130                 135                 140

Thr Val
145

<210>  19
<211>  369
<212>  DNA
<213>  Homo sapiens

<220>
<221>  CDS
<222>  (1)..(369)
<223>  new1

<400>  19
ggg gga ggc ttg ctc aag cca gga ggg tcc ctg aga ctc tcc tgt gta      48
Gly Gly Gly Leu Leu Lys Pro Gly Gly Ser Leu Arg Leu Ser Cys Val
1               5                   10                  15

gcc tct gga ttc agc ata agc gac ttc tac atg agt tgg atc cgc cag      96
Ala Ser Gly Phe Ser Ile Ser Asp Phe Tyr Met Ser Trp Ile Arg Gln
            20                  25                  30

gct cca ggg aaa gga ctg gag tgg atc tca tac ctc agt ggt ggc agt      144
Ala Pro Gly Lys Gly Leu Glu Trp Ile Ser Tyr Leu Ser Gly Gly Ser
        35                  40                  45

act tac agg agc cac gca gac tct ggg aag ggc cga ttc acc atc tcc      192
Thr Tyr Arg Ser His Ala Asp Ser Gly Lys Gly Arg Phe Thr Ile Ser
    50                  55                  60

aga gac aac gcc aag aat tca ctg ttt ttg caa atg agt agc ctg gga      240
Arg Asp Asn Ala Lys Asn Ser Leu Phe Leu Gln Met Ser Ser Leu Gly

65                  70                  75                  80

gtc gag gac acg gcc gtg tat ttt tgt gcg agg cat gtg gga gtg gcg       288
Val Glu Asp Thr Ala Val Tyr Phe Cys Ala Arg His Val Gly Val Ala
                85                  90                  95

act gcc ttt gat atc tgg ggc caa ggg aca gtg gtc act gtc tcc tca       336
Thr Ala Phe Asp Ile Trp Gly Gln Gly Thr Val Val Thr Val Ser Ser
            100                 105                 110

gcc tcc acc aag ggc cca tcg gtc ttc ccc ctg                           369
Ala Ser Thr Lys Gly Pro Ser Val Phe Pro Leu
        115                 120

<210>  20
<211>  123
<212>  PRT
<213>  Homo sapiens

<400>  20

Gly Gly Gly Leu Leu Lys Pro Gly Gly Ser Leu Arg Leu Ser Cys Val
1               5                   10                  15

Ala Ser Gly Phe Ser Ile Ser Asp Phe Tyr Met Ser Trp Ile Arg Gln
            20                  25                  30

Ala Pro Gly Lys Gly Leu Glu Trp Ile Ser Tyr Leu Ser Gly Gly Ser
        35                  40                  45

Thr Tyr Arg Ser His Ala Asp Ser Gly Lys Gly Arg Phe Thr Ile Ser
    50                  55                  60

Arg Asp Asn Ala Lys Asn Ser Leu Phe Leu Gln Met Ser Ser Leu Gly
65                  70                  75                  80

Val Glu Asp Thr Ala Val Tyr Phe Cys Ala Arg His Val Gly Val Ala
                85                  90                  95

Thr Ala Phe Asp Ile Trp Gly Gln Gly Thr Val Val Thr Val Ser Ser
            100                 105                 110

Ala Ser Thr Lys Gly Pro Ser Val Phe Pro Leu
        115                 120

<210>  21
<211>  336
<212>  DNA
<213>  Homo sapiens

<220>
<221>  CDS
<222>  (1)..(336)
<223>  new2

<400>  21
ggg gga ggc ttg gtc gag cct gga ggg tcc ctg aga ctc tcc tgt gaa        48
Gly Gly Gly Leu Val Glu Pro Gly Gly Ser Leu Arg Leu Ser Cys Glu
1               5                   10                  15

gcc act gga ttc acc ttc agt gac tac tac atg agt tgg gtc cgc cag          96
Ala Thr Gly Phe Thr Phe Ser Asp Tyr Tyr Met Ser Trp Val Arg Gln
            20                  25                  30

gct cct ggg aag ggg ctg gaa tgg att gca tac att agt act ggt agt         144
Ala Pro Gly Lys Gly Leu Glu Trp Ile Ala Tyr Ile Ser Thr Gly Ser
        35                  40                  45

agt tac ata aat tat gca gac tct aag aag ggc cga ttc acc atc tcc         192
Ser Tyr Ile Asn Tyr Ala Asp Ser Lys Lys Gly Arg Phe Thr Ile Ser
    50                  55                  60

aga aac aac gcc aag aac tca ctg tat ctg caa ctg aac agc ctg aga         240
Arg Asn Asn Ala Lys Asn Ser Leu Tyr Leu Gln Leu Asn Ser Leu Arg
65                  70                  75                  80

gtc gac gac acg gcc gtg tat tac tgt gcg aga tcg aca cag agt ttc         288
Val Asp Asp Thr Ala Val Tyr Tyr Cys Ala Arg Ser Thr Gln Ser Phe
                85                  90                  95

ggg ttg tta tta ccc ctc gtc ctc ttt gac cac tgg ggc cag gga acc         336
Gly Leu Leu Leu Pro Leu Val Leu Phe Asp His Trp Gly Gln Gly Thr
            100                 105                 110


<210>  22
<211>  112
<212>  PRT
<213>  Homo sapiens

<400>  22

Gly Gly Gly Leu Val Glu Pro Gly Gly Ser Leu Arg Leu Ser Cys Glu
1               5                   10                  15


Ala Thr Gly Phe Thr Phe Ser Asp Tyr Tyr Met Ser Trp Val Arg Gln
            20                  25                  30


Ala Pro Gly Lys Gly Leu Glu Trp Ile Ala Tyr Ile Ser Thr Gly Ser
        35                  40                  45


Ser Tyr Ile Asn Tyr Ala Asp Ser Lys Lys Gly Arg Phe Thr Ile Ser
    50                  55                  60


Arg Asn Asn Ala Lys Asn Ser Leu Tyr Leu Gln Leu Asn Ser Leu Arg
65                  70                  75                  80


Val Asp Asp Thr Ala Val Tyr Tyr Cys Ala Arg Ser Thr Gln Ser Phe
                85                  90                  95


Gly Leu Leu Leu Pro Leu Val Leu Phe Asp His Trp Gly Gln Gly Thr
            100                 105                 110


<210>  23
<211>  369
<212>  DNA
<213>  Homo sapiens


<220>
<221>  CDS
<222>  (1)..(369)
<223>  new3

<400>  23
ggg gga ggc ttg gta cag cct ggg ggg tcc ctg aga ctc tcc tgt gca        48
Gly Gly Gly Leu Val Gln Pro Gly Gly Ser Leu Arg Leu Ser Cys Ala
1               5                   10                  15

gcc tct gga tcc acc tta atc aac tat gcc atg agc tgg gtc cgc cag        96
Ala Ser Gly Ser Thr Leu Ile Asn Tyr Ala Met Ser Trp Val Arg Gln
            20                  25                  30

gct cca ggg aag ggg ctg gag tgg gtc tca gtt att agt gga act ggt       144
Ala Pro Gly Lys Gly Leu Glu Trp Val Ser Val Ile Ser Gly Thr Gly
        35                  40                  45

gtt ggc aca tac tac gca gac tcc gtg agg ggc cgg ttc acc atc tcc       192
Val Gly Thr Tyr Tyr Ala Asp Ser Val Arg Gly Arg Phe Thr Ile Ser
    50                  55                  60

aga gac gat tcc aac aac acg gtg gat ctg caa atg aat agc ctg aga       240
Arg Asp Asp Ser Asn Asn Thr Val Asp Leu Gln Met Asn Ser Leu Arg
65                  70                  75                  80

gcc gag gac acg gcc gta tat tac tgt gcg aaa gat ttc caa gtc ttc       288
Ala Glu Asp Thr Ala Val Tyr Tyr Cys Ala Lys Asp Phe Gln Val Phe
                85                  90                  95

ggt gac tac att tct cta ggc tat tgg ggc cag gga atc ctg gtc acc       336
Gly Asp Tyr Ile Ser Leu Gly Tyr Trp Gly Gln Gly Ile Leu Val Thr
            100                 105                 110

gtc gcc tca gcc tcc acc aag ggc cca tcg gtc                           369
Val Ala Ser Ala Ser Thr Lys Gly Pro Ser Val
        115                 120


<210>  24
<211>  123
<212>  PRT
<213>  Homo sapiens

<400>  24

Gly Gly Gly Leu Val Gln Pro Gly Gly Ser Leu Arg Leu Ser Cys Ala
1               5                   10                  15


Ala Ser Gly Ser Thr Leu Ile Asn Tyr Ala Met Ser Trp Val Arg Gln
            20                  25                  30


Ala Pro Gly Lys Gly Leu Glu Trp Val Ser Val Ile Ser Gly Thr Gly
        35                  40                  45


Val Gly Thr Tyr Tyr Ala Asp Ser Val Arg Gly Arg Phe Thr Ile Ser
    50                  55                  60


Arg Asp Asp Ser Asn Asn Thr Val Asp Leu Gln Met Asn Ser Leu Arg
65                  70                  75                  80


Ala Glu Asp Thr Ala Val Tyr Tyr Cys Ala Lys Asp Phe Gln Val Phe
                85                  90                  95


Gly Asp Tyr Ile Ser Leu Gly Tyr Trp Gly Gln Gly Ile Leu Val Thr
            100                 105                 110

Val Ala Ser Ala Ser Thr Lys Gly Pro Ser Val
        115                 120

<210>  25
<211>  458
<212>  DNA
<213>  Homo sapiens

<220>
<221>  CDS
<222>  (1)..(456)
<223>  PL12HC11A

<400>  25
ggg gga ggc ttg gca cag cct ggg ggg tcc ctg aga ctc tcc tgt gca        48
Gly Gly Gly Leu Ala Gln Pro Gly Gly Ser Leu Arg Leu Ser Cys Ala
1               5                   10                  15

gcc tct gga ttc acc ttt agc agc cat ggc atg agc tgg gtc cgc cag        96
Ala Ser Gly Phe Thr Phe Ser Ser His Gly Met Ser Trp Val Arg Gln
            20                  25                  30

gct cca ggg aag ggg ctg gag tgg gtc tca gct att agt ggg agt ggt       144
Ala Pro Gly Lys Gly Leu Glu Trp Val Ser Ala Ile Ser Gly Ser Gly
        35                  40                  45

ggt aac act tac tac gca gac tcc gtg aag ggc cgg ttc acc atc tcc       192
Gly Asn Thr Tyr Tyr Ala Asp Ser Val Lys Gly Arg Phe Thr Ile Ser
    50                  55                  60

aga gac att tcc aag aac acg ctg tat ctg caa atg aac agc ctg aga       240
Arg Asp Ile Ser Lys Asn Thr Leu Tyr Leu Gln Met Asn Ser Leu Arg
65                  70                  75                  80

gcc gaa gac acg gcc cta tat tac tgt gcg aga ggg ggc gcc tat ggt       288
Ala Glu Asp Thr Ala Leu Tyr Tyr Cys Ala Arg Gly Gly Ala Tyr Gly
                85                  90                  95

tcg ggg agt tat aag tac tgg ggc cag gga acc ctg gtc acc gtc tcc       336
Ser Gly Ser Tyr Lys Tyr Trp Gly Gln Gly Thr Leu Val Thr Val Ser
            100                 105                 110

tca gcc tcc acc aag ggc cca tcg gtc ttc ccc ctg gca ccc tcc tcc       384
Ser Ala Ser Thr Lys Gly Pro Ser Val Phe Pro Leu Ala Pro Ser Ser
        115                 120                 125

aag agc acc tct ggg ggc aca gcg gcc ctg ggc tgc ctg gtc aag gac       432
Lys Ser Thr Ser Gly Gly Thr Ala Ala Leu Gly Cys Leu Val Lys Asp
    130                 135                 140

tac ttc ccc gaa ccg gtg acg gtg tc                                    458
Tyr Phe Pro Glu Pro Val Thr Val
145                 150

<210>  26
<211>  152
<212>  PRT
<213>  Homo sapiens

<400>  26

Gly Gly Gly Leu Ala Gln Pro Gly Gly Ser Leu Arg Leu Ser Cys Ala
1               5                   10                  15

Ala Ser Gly Phe Thr Phe Ser Ser His Gly Met Ser Trp Val Arg Gln
            20                  25                  30

Ala Pro Gly Lys Gly Leu Glu Trp Val Ser Ala Ile Ser Gly Ser Gly
        35                  40                  45

Gly Asn Thr Tyr Tyr Ala Asp Ser Val Lys Gly Arg Phe Thr Ile Ser
    50                  55                  60

Arg Asp Ile Ser Lys Asn Thr Leu Tyr Leu Gln Met Asn Ser Leu Arg
65                  70                  75                  80

Ala Glu Asp Thr Ala Leu Tyr Tyr Cys Ala Arg Gly Gly Ala Tyr Gly
                85                  90                  95

Ser Gly Ser Tyr Lys Tyr Trp Gly Gln Gly Thr Leu Val Thr Val Ser
            100                 105                 110

Ser Ala Ser Thr Lys Gly Pro Ser Val Phe Pro Leu Ala Pro Ser Ser
        115                 120                 125

Lys Ser Thr Ser Gly Gly Thr Ala Ala Leu Gly Cys Leu Val Lys Asp
    130                 135                 140

Tyr Phe Pro Glu Pro Val Thr Val
145                 150

<210>  27
<211>  473
<212>  DNA
<213>  Homo sapiens

<220>
<221>  CDS
<222>  (1)..(471)
<223>  PL12HC28

<400>  27
ggg gga ggc gtg gtc cag cct ggg agg tcc ctg aga ctc tcc tgt gca        48
Gly Gly Gly Val Val Gln Pro Gly Arg Ser Leu Arg Leu Ser Cys Ala
1               5                   10                  15

gcc tct gga ttc acc ttc agt agc tat gac atg cac tgg gtc cgc cag        96
Ala Ser Gly Phe Thr Phe Ser Ser Tyr Asp Met His Trp Val Arg Gln
            20                  25                  30

gct cca ggc aag ggg ctg gag tgg gtg gca att ata ttg gat gat gga       144
Ala Pro Gly Lys Gly Leu Glu Trp Val Ala Ile Ile Leu Asp Asp Gly
        35                  40                  45

agt aat aaa tac tat gca gcc tcc gtg aag ggc cga ttc acc atc tcc       192
Ser Asn Lys Tyr Tyr Ala Ala Ser Val Lys Gly Arg Phe Thr Ile Ser
    50                  55                  60

aga gac aat tcc aag aac acg ctg tat ctg caa atg aac agc ctg aga       240
Arg Asp Asn Ser Lys Asn Thr Leu Tyr Leu Gln Met Asn Ser Leu Arg
65                  70                  75                  80

gct gag gac acg gct gtg tat tac tgt gcg aaa gtg cga ata ggg aag       288
Ala Glu Asp Thr Ala Val Tyr Tyr Cys Ala Lys Val Arg Ile Gly Lys
                85                  90                  95

gtc aat aag gtc aat aag tcc tac ttt gac tcc tgg ggc cag gga acc        336
Val Asn Lys Val Asn Lys Ser Tyr Phe Asp Ser Trp Gly Gln Gly Thr
            100                 105                 110

ctg gtc acc gtc tcc tca gcc tcc acc aag ggc cca tcg gtc ttc ccc        384
Leu Val Thr Val Ser Ser Ala Ser Thr Lys Gly Pro Ser Val Phe Pro
        115                 120                 125

ctg gca ccc tcc tcc aag agc acc tct ggg ggc aca gcg gcc ctg ggc        432
Leu Ala Pro Ser Ser Lys Ser Thr Ser Gly Gly Thr Ala Ala Leu Gly
    130                 135                 140

tgc ctg gtc aag gac tac ttc ccc gaa ccg gtg acg gtg tc                 473
Cys Leu Val Lys Asp Tyr Phe Pro Glu Pro Val Thr Val
145                 150                 155


<210>  28
<211>  157
<212>  PRT
<213>  Homo sapiens

<400>  28

Gly Gly Gly Val Val Gln Pro Gly Arg Ser Leu Arg Leu Ser Cys Ala
1               5                   10                  15

Ala Ser Gly Phe Thr Phe Ser Ser Tyr Asp Met His Trp Val Arg Gln
            20                  25                  30

Ala Pro Gly Lys Gly Leu Glu Trp Val Ala Ile Ile Leu Asp Asp Gly
        35                  40                  45

Ser Asn Lys Tyr Tyr Ala Ala Ser Val Lys Gly Arg Phe Thr Ile Ser
    50                  55                  60

Arg Asp Asn Ser Lys Asn Thr Leu Tyr Leu Gln Met Asn Ser Leu Arg
65                  70                  75                  80

Ala Glu Asp Thr Ala Val Tyr Tyr Cys Ala Lys Val Arg Ile Gly Lys
                85                  90                  95

Val Asn Lys Val Asn Lys Ser Tyr Phe Asp Ser Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val
145                 150                 155


<210>  29
<211>  377
<212>  DNA
<213>  Homo sapiens

<220>
<221>  CDS
<222>  (1)..(375)
<223>  PL12LC18

<400>  29
gcc atg gcc gag ctc acg cag tct cca ggc acc ctg tct ttg tct cca        48
Ala Met Ala Glu Leu Thr Gln Ser Pro Gly Thr Leu Ser Leu Ser Pro
1               5                   10                  15

ggg gaa aga gtc acc ctc tcc tgc agg gcc agc cag agc gtt agt agc        96
Gly Glu Arg Val Thr Leu Ser Cys Arg Ala Ser Gln Ser Val Ser Ser
            20                  25                  30

aac tac tta acc tgg tac cag cag aaa cct ggc cag gct ccc agg ctc       144
Asn Tyr Leu Thr Trp Tyr Gln Gln Lys Pro Gly Gln Ala Pro Arg Leu
        35                  40                  45

ctc atc tat ggt gca tcc aga agg gcc act ggc atc cca gac agg ttc       192
Leu Ile Tyr Gly Ala Ser Arg Arg Ala Thr Gly Ile Pro Asp Arg Phe
    50                  55                  60

agt ggc agt ggg tct ggg acc gac ttc act ctc acc ata agc aga ctg       240
Ser Gly Ser Gly Ser Gly Thr Asp Phe Thr Leu Thr Ile Ser Arg Leu
65                  70                  75                  80

gag cct gaa gat ttt gca gtt tat tac tgt caa cat tat ggt agc tca       288
Glu Pro Glu Asp Phe Ala Val Tyr Tyr Cys Gln His Tyr Gly Ser Ser
                85                  90                  95

cct cca ttc cct ttc ggc cct ggg acc aaa gtg gat gtc aaa cga act       336
Pro Pro Phe Pro Phe Gly Pro Gly Thr Lys Val Asp Val Lys Arg Thr
            100                 105                 110

gtg gct gca cca tct gtc ttc atc ttc ccg cca tct gat ga                377
Val Ala Ala Pro Ser Val Phe Ile Phe Pro Pro Ser Asp
        115                 120                 125


<210>  30
<211>  125
<212>  PRT
<213>  Homo sapiens

<400>  30

Ala Met Ala Glu Leu Thr Gln Ser Pro Gly Thr Leu Ser Leu Ser Pro
1               5                   10                  15


Gly Glu Arg Val Thr Leu Ser Cys Arg Ala Ser Gln Ser Val Ser Ser
            20                  25                  30


Asn Tyr Leu Thr Trp Tyr Gln Gln Lys Pro Gly Gln Ala Pro Arg Leu
        35                  40                  45


Leu Ile Tyr Gly Ala Ser Arg Arg Ala Thr Gly Ile Pro Asp Arg Phe
    50                  55                  60


Ser Gly Ser Gly Ser Gly Thr Asp Phe Thr Leu Thr Ile Ser Arg Leu
65                  70                  75                  80


Glu Pro Glu Asp Phe Ala Val Tyr Tyr Cys Gln His Tyr Gly Ser Ser
                85                  90                  95

Pro Pro Phe Pro Phe Gly Pro Gly Thr Lys Val Asp Val Lys Arg Thr
            100                 105                 110

Val Ala Ala Pro Ser Val Phe Ile Phe Pro Pro Ser Asp
        115                 120                 125

<210>  31
<211>  374
<212>  DNA
<213>  Homo sapiens

<220>
<221>  CDS
<222>  (1)..(372)
<223>  PL12LC5

<400>  31
gcc atg gcc gag ctc acg cag tct cca ggc acc ctg tct ttg tct cca      48
Ala Met Ala Glu Leu Thr Gln Ser Pro Gly Thr Leu Ser Leu Ser Pro
1               5                   10                  15

ggg gaa aga gcc acc ctc tcc tgc agg gcc agt cag agc att cgc agc      96
Gly Glu Arg Ala Thr Leu Ser Cys Arg Ala Ser Gln Ser Ile Arg Ser
            20                  25                  30

aac ttc tta gcc tgg tac cag cag aaa cct ggc cag gct ccc agg ctc      144
Asn Phe Leu Ala Trp Tyr Gln Gln Lys Pro Gly Gln Ala Pro Arg Leu
        35                  40                  45

ctc atc ttt ggt gca tcg aac agg gcc act ggc atc cca gac agg ttc      192
Leu Ile Phe Gly Ala Ser Asn Arg Ala Thr Gly Ile Pro Asp Arg Phe
    50                  55                  60

agt ggc agt ggg tct ggg aca gac ttc act ctc acc atc agt aga ctg      240
Ser Gly Ser Gly Ser Gly Thr Asp Phe Thr Leu Thr Ile Ser Arg Leu
65                  70                  75                  80

gag cct gaa gat ttt gcg gtt tat tac tgt cag cag tat agt agc tca      288
Glu Pro Glu Asp Phe Ala Val Tyr Tyr Cys Gln Gln Tyr Ser Ser Ser
                85                  90                  95

ccg gac act ttt ggc cag ggg acc aag ctg gag atc aaa cga act gtg      336
Pro Asp Thr Phe Gly Gln Gly Thr Lys Leu Glu Ile Lys Arg Thr Val
            100                 105                 110

gct gca cca tct gtc ttc atc ttc ccg cca tct gat ga                   374
Ala Ala Pro Ser Val Phe Ile Phe Pro Pro Ser Asp
        115                 120

<210>  32
<211>  124
<212>  PRT
<213>  Homo sapiens

<400>  32

Ala Met Ala Glu Leu Thr Gln Ser Pro Gly Thr Leu Ser Leu Ser Pro
1               5                   10                  15

Gly Glu Arg Ala Thr Leu Ser Cys Arg Ala Ser Gln Ser Ile Arg Ser
            20                  25                  30

Asn Phe Leu Ala Trp Tyr Gln Gln Lys Pro Gly Gln Ala Pro Arg Leu
        35                  40                  45

Leu Ile Phe Gly Ala Ser Asn Arg Ala Thr Gly Ile Pro Asp Arg Phe
    50                  55                  60

Ser Gly Ser Gly Ser Gly Thr Asp Phe Thr Leu Thr Ile Ser Arg Leu
65                  70                  75                  80

Glu Pro Glu Asp Phe Ala Val Tyr Tyr Cys Gln Gln Tyr Ser Ser Ser
                85                  90                  95

Pro Asp Thr Phe Gly Gln Gly Thr Lys Leu Glu Ile Lys Arg Thr Val
            100                 105                 110

Ala Ala Pro Ser Val Phe Ile Phe Pro Pro Ser Asp
        115                 120

<210>  33
<211>  371
<212>  DNA
<213>  Homo sapiens

<220>
<221>  CDS
<222>  (1)..(369)
<223>  PL12LC26

<400>  33
gcc atg gcc gag ctc acc cag tct cca tcc tcc ctg tct gca tct gta        48
Ala Met Ala Glu Leu Thr Gln Ser Pro Ser Ser Leu Ser Ala Ser Val
1               5                   10                  15

gga gac aga gtc acc gtc act tgc cgg gca agt cag acc att gcc aac        96
Gly Asp Arg Val Thr Val Thr Cys Arg Ala Ser Gln Thr Ile Ala Asn
            20                  25                  30

tat tta aat tgg tat cag caa aaa cca ggg aaa gcc cct aac ctc ctg       144
Tyr Leu Asn Trp Tyr Gln Gln Lys Pro Gly Lys Ala Pro Asn Leu Leu
        35                  40                  45

atc caa gct gct tcc act ttg caa ggt ggg gtc cca tca agg ttc agt       192
Ile Gln Ala Ala Ser Thr Leu Gln Gly Gly Val Pro Ser Arg Phe Ser
    50                  55                  60

ggc agt cga tct ggg aca gat ttc act ctc acc atc acc agt ctg cag       240
Gly Ser Arg Ser Gly Thr Asp Phe Thr Leu Thr Ile Thr Ser Leu Gln
65                  70                  75                  80

cct gag gat ttt gca act tac ttc tgt caa cag agt ttc agc gcc ccc       288
Pro Glu Asp Phe Ala Thr Tyr Phe Cys Gln Gln Ser Phe Ser Ala Pro
                85                  90                  95

tgg acg ttc ggc caa ggg acc aaa gtg gaa atc aaa cga act gtg gct       336
Trp Thr Phe Gly Gln Gly Thr Lys Val Glu Ile Lys Arg Thr Val Ala
            100                 105                 110

gca cca tct gtc ttc atc ttc ccg cca tct gat ga                        371
Ala Pro Ser Val Phe Ile Phe Pro Pro Ser Asp
        115                 120

<210>  34
<211>  123
<212>  PRT

<213>  Homo sapiens

<400>  34

Ala Met Ala Glu Leu Thr Gln Ser Pro Ser Ser Leu Ser Ala Ser Val
1               5                   10                  15

Gly Asp Arg Val Thr Val Thr Cys Arg Ala Ser Gln Thr Ile Ala Asn
            20                  25                  30

Tyr Leu Asn Trp Tyr Gln Gln Lys Pro Gly Lys Ala Pro Asn Leu Leu
        35                  40                  45

Ile Gln Ala Ala Ser Thr Leu Gln Gly Gly Val Pro Ser Arg Phe Ser
    50                  55                  60

Gly Ser Arg Ser Gly Thr Asp Phe Thr Leu Thr Ile Thr Ser Leu Gln
65                  70                  75                  80

Pro Glu Asp Phe Ala Thr Tyr Phe Cys Gln Gln Ser Phe Ser Ala Pro
                85                  90                  95

Trp Thr Phe Gly Gln Gly Thr Lys Val Glu Ile Lys Arg Thr Val Ala
            100                 105                 110

Ala Pro Ser Val Phe Ile Phe Pro Pro Ser Asp
        115                 120

<210>  35
<211>  30
<212>  DNA
<213>  Homo sapiens

<220>
<221>  CDS
<222>  (1)..(30)
<223>  Fab7816-HC2-CDR1

<400>  35
ggt ggc tcc atc ggc agt ggt tct tac tcc                              30
Gly Gly Ser Ile Gly Ser Gly Ser Tyr Ser
1               5                   10

<210>  36
<211>  10
<212>  PRT
<213>  Homo sapiens

<400>  36

Gly Gly Ser Ile Gly Ser Gly Ser Tyr Ser
1               5                   10

<210>  37
<211>  21
<212>  DNA
<213>  Homo sapiens

<220>
<221>  CDS
<222>  (1)..(21)
<223>  Fab7816-HC2-CDR2

<400>  37
atc tct gac agt ggg aac acc                                          21
Ile Ser Asp Ser Gly Asn Thr
1               5


<210>  38
<211>  7
<212>  PRT
<213>  Homo sapiens

<400>  38

Ile Ser Asp Ser Gly Asn Thr
1               5


<210>  39
<211>  45
<212>  DNA
<213>  Homo sapiens


<220>
<221>  CDS
<222>  (1)..(45)
<223>  Fab7816-HC2-CDR3

<400>  39
tgt gcg aga ggg aga ggt att ttg act ggt ctc ttt gac tat tgg          45
Cys Ala Arg Gly Arg Gly Ile Leu Thr Gly Leu Phe Asp Tyr Trp
1               5                   10                  15


<210>  40
<211>  15
<212>  PRT
<213>  Homo sapiens

<400>  40

Cys Ala Arg Gly Arg Gly Ile Leu Thr Gly Leu Phe Asp Tyr Trp
1               5                   10                  15


<210>  41
<211>  36
<212>  DNA
<213>  Homo sapiens


<220>
<221>  CDS
<222>  (1)..(36)
<223>  Fab7816-LC4-CDR1

<400>  41
cag agt gtt ttg gac aac tcc aac cat aag aac tcc                      36
Gln Ser Val Leu Asp Asn Ser Asn His Lys Asn Ser
1               5                   10


<210>  42
<211>  12
<212>  PRT

<213>  Homo sapiens

<400>  42

Gln Ser Val Leu Asp Asn Ser Asn His Lys Asn Ser
1               5                   10


<210>  43
<211>  33
<212>  DNA
<213>  Homo sapiens


<220>
<221>  CDS
<222>  (1)..(33)
<223>  Fab7816-LC4-CDR3

<400>  43
acc ttc tgt cag caa tat tat agt act ccg tgg                          33
Thr Phe Cys Gln Gln Tyr Tyr Ser Thr Pro Trp
1               5                   10


<210>  44
<211>  11
<212>  PRT
<213>  Homo sapiens

<400>  44

Thr Phe Cys Gln Gln Tyr Tyr Ser Thr Pro Trp
1               5                   10


<210>  45
<211>  27
<212>  DNA
<213>  Homo sapiens


<220>
<221>  CDS
<222>  (1)..(27)
<223>  Fab5Lcx-HC2-CDR1

<400>  45
ggt ggc tcc atc agc agt agt aac tgg                                  27
Gly Gly Ser Ile Ser Ser Ser Asn Trp
1               5


<210>  46
<211>  9
<212>  PRT
<213>  Homo sapiens

<400>  46

Gly Gly Ser Ile Ser Ser Ser Asn Trp
1               5


<210>  47
<211>  21
<212>  DNA
<213>  Homo sapiens

<220>
<221>  CDS
<222>  (1)..(21)
<223>  Fab5Lcx-HC2-CDR2

<400>  47
atc gat cat agt ggg act acc                                                21
Ile Asp His Ser Gly Thr Thr
1               5


<210>  48
<211>  7
<212>  PRT
<213>  Homo sapiens

<400>  48

Ile Asp His Ser Gly Thr Thr
1               5


<210>  49
<211>  42
<212>  DNA
<213>  Homo sapiens


<220>
<221>  CDS
<222>  (1)..(42)
<223>  Fab5Lcx-HC2-CDR3

<400>  49
tgt gcg aga gga gca aag gat aac tgg gga ttc gac tac tgg                    42
Cys Ala Arg Gly Ala Lys Asp Asn Trp Gly Phe Asp Tyr Trp
1               5                   10


<210>  50
<211>  14
<212>  PRT
<213>  Homo sapiens

<400>  50

Cys Ala Arg Gly Ala Lys Asp Asn Trp Gly Phe Asp Tyr Trp
1               5                   10


<210>  51
<211>  33
<212>  DNA
<213>  Homo sapiens


<220>
<221>  CDS
<222>  (1)..(33)
<223>  Fab5Lcx-LC8-CDR3

<400>  51
tgt caa cac gat tac aat gac cct cga act ttc                                33
Cys Gln His Asp Tyr Asn Asp Pro Arg Thr Phe
1               5                   10


<210>  52
<211>  11
<212>  PRT

<213>  Homo sapiens

<400>  52

Cys Gln His Asp Tyr Asn Asp Pro Arg Thr Phe
1               5                   10


<210>  53
<211>  24
<212>  DNA
<213>  Homo sapiens


<220>
<221>  CDS
<222>  (1)..(24)
<223>  Fab1630-HC10-CDR1

<400>  53
gga ttc act ttc agt aac ggc tgg                                          24
Gly Phe Thr Phe Ser Asn Gly Trp
1               5


<210>  54
<211>  8
<212>  PRT
<213>  Homo sapiens

<400>  54

Gly Phe Thr Phe Ser Asn Gly Trp
1               5


<210>  55
<211>  30
<212>  DNA
<213>  Homo sapiens


<220>
<221>  CDS
<222>  (1)..(30)
<223>  Fab1630-HC10-CDR2

<400>  55
att aga agc aac ccc gac ggt ggc aca aca                                  30
Ile Arg Ser Asn Pro Asp Gly Gly Thr Thr
1               5                   10


<210>  56
<211>  10
<212>  PRT
<213>  Homo sapiens

<400>  56

Ile Arg Ser Asn Pro Asp Gly Gly Thr Thr
1               5                   10


<210>  57
<211>  57
<212>  DNA
<213>  Homo sapiens

<220>
<221>  CDS
<222>  (1)..(57)
<223>  Fab1630-HC10-CDR3

<400>  57
tgc atc aca gat cgg ggt gac tgg aag tgg ggg gtc cct agg gac ctc        48
Cys Ile Thr Asp Arg Gly Asp Trp Lys Trp Gly Val Pro Arg Asp Leu
1               5                   10                  15

acc tac tgg                                                            57
Thr Tyr Trp

<210>  58
<211>  19
<212>  PRT
<213>  Homo sapiens

<400>  58

Cys Ile Thr Asp Arg Gly Asp Trp Lys Trp Gly Val Pro Arg Asp Leu
1               5                   10                  15

Thr Tyr Trp

<210>  59
<211>  21
<212>  DNA
<213>  Homo sapiens

<220>
<221>  CDS
<222>  (1)..(21)
<223>  Fab1630-LC12-CDR1

<400>  59
aac tac cag agt gtc gac agc                                            21
Asn Tyr Gln Ser Val Asp Ser
1               5

<210>  60
<211>  7
<212>  PRT
<213>  Homo sapiens

<400>  60

Asn Tyr Gln Ser Val Asp Ser
1               5

<210>  61
<211>  33
<212>  DNA
<213>  Homo sapiens

<220>
<221>  CDS
<222>  (1)..(33)
<223>  Fab1630-LC12-CDR3

<400>  61

tgt cag cag tat ctt agc ccg ccg atc acc ttc                                    33
Cys Gln Gln Tyr Leu Ser Pro Pro Ile Thr Phe
1               5                   10


<210>  62
<211>  11
<212>  PRT
<213>  Homo sapiens

<400>  62

Cys Gln Gln Tyr Leu Ser Pro Pro Ile Thr Phe
1               5                   10


<210>  63
<211>  24
<212>  DNA
<213>  Homo sapiens


<220>
<221>  CDS
<222>  (1)..(24)
<223>  Fab248-HC14-CDR1

<400>  63
gga ttc acc ttc agt gac tac tac                                                24
Gly Phe Thr Phe Ser Asp Tyr Tyr
1               5


<210>  64
<211>  8
<212>  PRT
<213>  Homo sapiens

<400>  64

Gly Phe Thr Phe Ser Asp Tyr Tyr
1               5


<210>  65
<211>  24
<212>  DNA
<213>  Homo sapiens


<220>
<221>  CDS
<222>  (1)..(24)
<223>  Fab248-HC14-CDR2

<400>  65
att agt agt ggt ggt gac acc ata                                                24
Ile Ser Ser Gly Gly Asp Thr Ile
1               5


<210>  66
<211>  8
<212>  PRT
<213>  Homo sapiens

<400>  66

Ile Ser Ser Gly Gly Asp Thr Ile
1               5

<210>  67
<211>  21
<212>  DNA
<213>  Homo sapiens


<220>
<221>  CDS
<222>  (1)..(21)
<223>  Fab248-HC14-CDR3

<400>  67
tgt gcg tgc cgt ggg gtc tgg                                                21
Cys Ala Cys Arg Gly Val Trp
1               5


<210>  68
<211>  7
<212>  PRT
<213>  Homo sapiens

<400>  68

Cys Ala Cys Arg Gly Val Trp
1               5


<210>  69
<211>  18
<212>  DNA
<213>  Homo sapiens


<220>
<221>  CDS
<222>  (1)..(18)
<223>  Fab248-LC16-CDR1

<400>  69
cag agt att agt ttc cac                                                    18
Gln Ser Ile Ser Phe His
1               5


<210>  70
<211>  6
<212>  PRT
<213>  Homo sapiens

<400>  70

Gln Ser Ile Ser Phe His
1               5


<210>  71
<211>  47
<212>  DNA
<213>  Artificial Sequence

<220>
<223>  primer


<220>
<221>  primer_bind
<222>  (1)..(47)

<223>  primer OmpK36NcoIFW

<400>  71
gcgctagtat gccatgggca tgaaagttaa agtactgtcc ctcctgg                47

<210>  72
<211>  45
<212>  DNA
<213>  Artificial Sequence

<220>
<223>  primer

<220>
<221>  protein_bind
<222>  (1)..(45)
<223>  primer OmpK36XhoIRW

<400>  72
gcgcttcctc gatacctcga gctagaactg gtaaaccagg cccag                  45

<210>  73
<211>  46
<212>  DNA
<213>  Artificial Sequence

<220>
<223>  primer

<220>
<221>  primer_bind
<222>  (1)..(46)
<223>  primer OmpK36BamHIFW

<400>  73
gcgctagtct gggatccgat gaaagttaaa gtactgtccc tcctgg                 46

<210>  74
<211>  20
<212>  DNA
<213>  Artificial Sequence

<220>
<223>  primer

<220>
<221>  primer_bind
<222>  (1)..(20)
<223>  primer T7FWpET

<400>  74
taatacgact cactataggg                                              20

<210>  75
<211>  30
<212>  DNA
<213>  Artificial Sequence

<220>
<223>  primer

<220>

<221>  primer_bind
<222>  (1)..(19)
<223>  primer T7termpET

<400>  75
ggtggctcca tcggcagtgg ttcttactcc                                        30


<210>  76
<211>  1098
<212>  DNA
<213>  Klebsiella pneumoniae


<220>
<221>  CDS
<222>  (1)..(1098)
<223>  OmpK36

<400>  76
atg aaa gtt aaa gta ctg tcc ctc ctg gta ccg gct ctg ctg gta gca       48
Met Lys Val Lys Val Leu Ser Leu Leu Val Pro Ala Leu Leu Val Ala
1               5                   10                  15

ggc gca gca aat gcg gct gaa att tat aac aaa gac ggc aac aaa tta       96
Gly Ala Ala Asn Ala Ala Glu Ile Tyr Asn Lys Asp Gly Asn Lys Leu
            20                  25                  30

gac ctg tac ggt aaa att gac ggt ctg cac tac ttc tct gac gac aag      144
Asp Leu Tyr Gly Lys Ile Asp Gly Leu His Tyr Phe Ser Asp Asp Lys
        35                  40                  45

agc gtc gac ggc gac cag acc tac atg cgt gta ggc gtg aaa ggc gaa      192
Ser Val Asp Gly Asp Gln Thr Tyr Met Arg Val Gly Val Lys Gly Glu
    50                  55                  60

acc cag atc aac gac cag ctg acc ggt tac ggc cag tgg gaa tac aac      240
Thr Gln Ile Asn Asp Gln Leu Thr Gly Tyr Gly Gln Trp Glu Tyr Asn
65                  70                  75                  80

gtt cag gcg aac aac act gaa agc tcc agc gat cag gca tgg act cgt      288
Val Gln Ala Asn Asn Thr Glu Ser Ser Ser Asp Gln Ala Trp Thr Arg
                85                  90                  95

ctg gca ttc gca ggc ctg aaa ttt ggc gac gcg ggc tct ttc gac tac      336
Leu Ala Phe Ala Gly Leu Lys Phe Gly Asp Ala Gly Ser Phe Asp Tyr
            100                 105                 110

ggt cgt aac tac ggc gta gta tac gtc gta acg tcc tgg acc gac gtt      384
Gly Arg Asn Tyr Gly Val Val Tyr Val Val Thr Ser Trp Thr Asp Val
        115                 120                 125

ctg ccg gaa ttc ggc ggc gac acc tac ggt tct gac aac ttc ctg cag      432
Leu Pro Glu Phe Gly Gly Asp Thr Tyr Gly Ser Asp Asn Phe Leu Gln
    130                 135                 140

tcc cgt gct aac ggc gtt gca acc tac cgt aac tct gat ttc ttc ggt      480
Ser Arg Ala Asn Gly Val Ala Thr Tyr Arg Asn Ser Asp Phe Phe Gly
145                 150                 155                 160

ctg gtt gac ggc ctg aac ttt gct ctg cag tat cag ggt aaa aac ggc      528
Leu Val Asp Gly Leu Asn Phe Ala Leu Gln Tyr Gln Gly Lys Asn Gly
                165                 170                 175

agc gtc agc ggc gaa ggc gcg acc aac aac ggt cgt ggt tgg agc aaa      576
Ser Val Ser Gly Glu Gly Ala Thr Asn Asn Gly Arg Gly Trp Ser Lys
            180                 185                 190

cag aac ggc gac ggc ttc ggc acc tct ctg acc tac gat att tgg gat      624
Gln Asn Gly Asp Gly Phe Gly Thr Ser Leu Thr Tyr Asp Ile Trp Asp

195                 200                 205

ggc atc agc gct ggt ttc gcg tac tct cac tcc aaa cgt acc gac gag       672
Gly Ile Ser Ala Gly Phe Ala Tyr Ser His Ser Lys Arg Thr Asp Glu
    210                 215                 220

cag aat agt gtt ccg gca ctg ggt cgt ggc gac aac gct gaa acc tac       720
Gln Asn Ser Val Pro Ala Leu Gly Arg Gly Asp Asn Ala Glu Thr Tyr
225                 230                 235                 240

acc ggt ggt ctg aaa tac gac gcc aac aac atc tac ctg gcc tct cag       768
Thr Gly Gly Leu Lys Tyr Asp Ala Asn Asn Ile Tyr Leu Ala Ser Gln
                245                 250                 255

tac acc cag acc tac aac gca act cgc gcc ggt tcc ctg ggc ttt gca       816
Tyr Thr Gln Thr Tyr Asn Ala Thr Arg Ala Gly Ser Leu Gly Phe Ala
            260                 265                 270

aac aaa gcg cag aac ttc gaa gtg gtt gct cag tac cag ttc gac ttc       864
Asn Lys Ala Gln Asn Phe Glu Val Val Ala Gln Tyr Gln Phe Asp Phe
        275                 280                 285

ggt ctg cgt ccg tct gtg gct tac ctg cag tct aaa ggt aag gat ctg       912
Gly Leu Arg Pro Ser Val Ala Tyr Leu Gln Ser Lys Gly Lys Asp Leu
    290                 295                 300

gag cgc ggc tac ggc gac cag gac atc ctg aaa tat gtt gac gtt ggc       960
Glu Arg Gly Tyr Gly Asp Gln Asp Ile Leu Lys Tyr Val Asp Val Gly
305                 310                 315                 320

gcg acc tac tac ttc aac aaa aac atg tcc acc tat gtt gac tac aaa      1008
Ala Thr Tyr Tyr Phe Asn Lys Asn Met Ser Thr Tyr Val Asp Tyr Lys
                325                 330                 335

atc aac ctg ctg gac gac aac agc ttc acc cgc aac gcc ggt atc tct      1056
Ile Asn Leu Leu Asp Asp Asn Ser Phe Thr Arg Asn Ala Gly Ile Ser
            340                 345                 350

acc gac gac gtg gtt gca ctg ggc ctg gtt tac cag ttc taa              1098
Thr Asp Asp Val Val Ala Leu Gly Leu Val Tyr Gln Phe
        355                 360                 365

<210>  77
<211>  365
<212>  PRT
<213>  Klebsiella pneumoniae

<400>  77

Met Lys Val Lys Val Leu Ser Leu Leu Val Pro Ala Leu Leu Val Ala
1               5                   10                  15

Gly Ala Ala Asn Ala Ala Glu Ile Tyr Asn Lys Asp Gly Asn Lys Leu
            20                  25                  30

Asp Leu Tyr Gly Lys Ile Asp Gly Leu His Tyr Phe Ser Asp Asp Lys
        35                  40                  45

Ser Val Asp Gly Asp Gln Thr Tyr Met Arg Val Gly Val Lys Gly Glu
    50                  55                  60

Thr Gln Ile Asn Asp Gln Leu Thr Gly Tyr Gly Gln Trp Glu Tyr Asn
65                  70                  75                  80

Val Gln Ala Asn Asn Thr Glu Ser Ser Ser Asp Gln Ala Trp Thr Arg
                85                  90                  95

Leu Ala Phe Ala Gly Leu Lys Phe Gly Asp Ala Gly Ser Phe Asp Tyr
            100                 105                 110

Gly Arg Asn Tyr Gly Val Val Tyr Val Val Thr Ser Trp Thr Asp Val
        115                 120                 125

Leu Pro Glu Phe Gly Gly Asp Thr Tyr Gly Ser Asp Asn Phe Leu Gln
    130                 135                 140

Ser Arg Ala Asn Gly Val Ala Thr Tyr Arg Asn Ser Asp Phe Phe Gly
145                 150                 155                 160

Leu Val Asp Gly Leu Asn Phe Ala Leu Gln Tyr Gln Gly Lys Asn Gly
                165                 170                 175

Ser Val Ser Gly Glu Gly Ala Thr Asn Asn Gly Arg Gly Trp Ser Lys
            180                 185                 190

Gln Asn Gly Asp Gly Phe Gly Thr Ser Leu Thr Tyr Asp Ile Trp Asp
        195                 200                 205

Gly Ile Ser Ala Gly Phe Ala Tyr Ser His Ser Lys Arg Thr Asp Glu
    210                 215                 220

Gln Asn Ser Val Pro Ala Leu Gly Arg Gly Asp Asn Ala Glu Thr Tyr
225                 230                 235                 240

Thr Gly Gly Leu Lys Tyr Asp Ala Asn Asn Ile Tyr Leu Ala Ser Gln
                245                 250                 255

Tyr Thr Gln Thr Tyr Asn Ala Thr Arg Ala Gly Ser Leu Gly Phe Ala
            260                 265                 270

Asn Lys Ala Gln Asn Phe Glu Val Val Ala Gln Tyr Gln Phe Asp Phe
        275                 280                 285

Gly Leu Arg Pro Ser Val Ala Tyr Leu Gln Ser Lys Gly Lys Asp Leu
    290                 295                 300

Glu Arg Gly Tyr Gly Asp Gln Asp Ile Leu Lys Tyr Val Asp Val Gly
305                 310                 315                 320

Ala Thr Tyr Tyr Phe Asn Lys Asn Met Ser Thr Tyr Val Asp Tyr Lys
                325                 330                 335

Ile Asn Leu Leu Asp Asp Asn Ser Phe Thr Arg Asn Ala Gly Ile Ser
            340                 345                 350

Thr Asp Asp Val Val Ala Leu Gly Leu Val Tyr Gln Phe
        355                 360                 365

<210>  78
<211>  36
<212>  DNA
<213>  Homo sapiens

<220>
<221>  CDS
<222>  (1)..(36)
<223>  Fab248LC-CDR3

<400>  78
tgt cag cag tat cat aac tgg cct ccc ctc act ttc                    36

<210>  79
<211>  12
<212>  PRT
<213>  Homo sapiens

<400>  79

## Description

The Acute Coronary Syndrome (also shortly referred to as ACS) is the manifestation of a plaque rupture in a coronary artery.

The rupture or the erosion of an atherosclerotic plaque, with the subsequent formation of thrombus and occlusion of the artery may cause myocardial infarction and unstable angina (see, for a general reference, "New insights into atherosclerotic plaque rupture" D.M. Braganza and M.R. Bennett, Postgrad. Med. J. 2001; 77; 94-98).

An atherosclerotic event begins as a subendothelial accumulation of lipid laden,monocyte derived foam cells and associated T cells which form a non-stenotic fatty streak. With progression, the lesion takes the form of an acellular core of cholesterol esters, bounded by an endothelialised fibrous cap containing smooth muscle cells (VMSC) and inflammatory cells (both macrophages and T lymphocytes). Also presented in the advanced lesions are new blood vessels and deposits of calcium hydroxyapatite may also appear in advanced lesions (see as a general reference, "Coronary disease: Atherogenesis: current understanding of the causes of atheroma" Peter L. Weissberg, Heart, 2000, 83: 247-252).

The extracellular lipid core of the plaque is composed of free cholesterol, cholesterol crystals and cholesterol esters derived from lipids that have infiltrated the arterial wall or derived from the dead foam cells. The lipid core may affect the plaque by causing stress to the shoulder regions of the plaque; in addition, the lipid core contains prothrombotic oxidized lipids and it is further impregnated with tissue factors derived from macrophages in which the lipid core materials are highly thrombogenic when exposed to circulating blood (see, for instance, Shah P. K., J. Am. College Cardiol, 2003:41 (Suppl. S) S15-S22).

The stability of the plaque depends also upon the vascular smooth muscle cell (SMC) content of the plaque, as they are capable of synthesising the structurally important collagen types I and III. In contrast, macrophages and other inflammatory cells may release matrix metallo-proteases (MMPs) which degrade collagen and extracellular matrix, thus potentially weakening the plaque (see, i.e. Braganza D.M. and Bennett M.R., Postgrad. Med. J. ,2001, 77: 94-98).

One possible cause of plaque formation is thought to be repeated injury to the endothelium caused by the four "major" risk factors: smoking, hypertension, diabetes and hyperlipidaemia (high level of LDL and low level of HDL). Endothelial dysfunction following injury, moreover, plays a crucial role in plaque initiation, as lipids may pass more easily from the bloodstream into the tunica intima.

Thus inflammation plays a key role in the development of the atherosclerotic disease.

In particular, as the inflammatory process becomes chronic, remodelling and formation of new intima is triggered. Furthermore, the interaction between lymphocytes and dendritic cells (DCs) within the neointima might be responsible for the development of a local immune response against exogenous and endogenous atherogenic antigens. Thus, atherosclerosis is considered to belong to the group of chronic inflammatory diseases for which whose development and complications are mainly due to the cellular components of the immune system. The chronic accumulation of monocytes/macrophages, smooth muscle cells, and T-lymphocytes in response to the accumulation and release of pro-inflammatory molecules within the arterial wall constitutes the hallmark of a developing atherosclerotic plaque.

The recent finding of the same authors of the present invention, that antibodies are produced within the plaque, as disclosed in WO2009/037297, seems to confirm the involvement of a local immune response (see also Burioni R. et al. J Immunol ., 2009. 183:2537-2544).

Although most of the antigenic stimuli that occur within atherosclerotic plaques come from modified self-molecules, the immune response triggered is remarkably similar to inflammatory reactions mounted against microbial organisms. Among the list of atherosclerosis-related antigens, ranging from oxidised low-density lipoproteins (oxLDL), heat shock proteins (HSP) to protein components of the extracellular matrix such as collagen and fibrinogen, transgelin 1 (also called SM22) has never been mentioned (see for a review: Milioti N. et al. Clin. Dev. Immunol., 2008, 2008:723539) even though transgelin 1 is known to be physiologically expressed in smooth muscle cells within the arterial wall, in particular as one of the earliest markers of smooth muscle cell differentiation due to its role in cytoskeleton organization (Assinder S. et al. lnt. J. Biochemistry & Cell Biology, 2009, 41:482-486).

Patents on transgelin uses are enlisted in the following: for example EP0914426 describes DNA sequences including a fragment of the sequence ahead of the coding portion of the murine SM22 protein gene. Said sequence and vectors have been proposed for treatment of coronary diseases, in particular restenosis. WO2007035451 provides methods of modulating angiogenesis in an individual, involving modulation of nicotinic acetylcholine receptor (nAChR), bFGF receptor, and VEGF receptor acting on the expression of, among others, TAGLN the gene encoding transgelin 1.

WO2007140972 proposes Transgelin-3 as a biomarker for Alzheimer's disease. US5837534 and US6015711 provide for sequences encoding the murine SM22 alpha promoter (arterial SMC-specific promoter), and gene transfer vectors containing the same, to target gene expression in arterial smooth muscle cells (gene therapy of the arterial vessel wall).

Thus, to our knowledge, although the expression of SM22 has been related to disorders involving smooth muscle cell activation, in particular in vascular remodelling, this protein has never been proposed as a relevant marker for atherosclerosis or predisposition to the development of this disease.

The present invention is based, in particular, on the new finding that transgelin and a category of bacterial antigens are immunologically related and, further, that the specificity of antibodies for these two antigens might be diagnostically relevant for patients suffering from Acute Coronary Syndrome or, even, during the development of this disease in the atherogenic process.

As a matter of fact, the role of foreign antigens, such as viruses and bacteria, in atherogenesis as causative or bystander participants in its development, has already been addressed and still is a controversial issue. To the best of our knowledge, however, neither Klebsiella nor outer membrane proteins have ever been associated with the development of atherosclerotic diseases, introducing another level of complexity to the analysis. Thus, in the present scenario, the disclosure of reagents with this new cross-reactivity entails to extremely valuable technical advancements, either in the diagnostic or research field related to atherosclerosis.

### SUMMARY OF THE INVENTION

The present invention refers to human antibodies able to immunologically recognize both human transgelin or fragments thereof and a protein with at least 50% similarity to OmpK36 (Outer membrane protein, Klebsiella, K36; GI: 295881594) or fragments thereof. Human transgelin is preferably transgelin 1 (Accession N° Q01995, GI:48255907).

Preferred antibodies comprise at least one of the following Heavy chains selected in the group consisting of: SEQIDNO:2, SEQIDNO:6, SEQIDNO:10, and at least one of the light chains selected in the group consisting of SEQIDNO:4, SEQIDNO:8, SEQIDNO:12. Even more preferably, the Heavy chain variable region consists of SEQIDNO:2 or SEQIDNO:10 and the Light chain variable region consists of: SEQIDNO:4 or SEQIDNO:12.

Even more preferably, the antibodies further recognize an antigen in the atherosclerotic plaque and are useful for the preparation of immunodiagnostic reagents or assays for atherogenic disorders, able to detect atherogenic ischemic or occlusive evolution in an arterial vessel, Acute Coronary syndrome and related cardiovascular diseases selected from the group consisting of: unstable angina, ST Elevation Myocardial Infarction (STEMI), nonSTEMI myocardial infarction), or intra-cerebral occlusive disease or peripheral artery occlusive diseases or a predisposition to any of those diseases. According to a preferred embodiment the method for detecting antibodies against an antigen in an atherosclerotic plaque comprises allowing an unknown biological sample to react with human transgelin or fragments thereof, optionally in competition with any of the antibodies according to the invention.

More preferably the immunoassay is a Western-blot or an ELISA.

According to a further embodiment the present invention also relates to transgelin-1 as a marker of the presence of an atherosclerotic plaque or of an atherogenic development within arteries.

### BRIEF DESCRIPTION OF THE DRAWINGS

**Figure 1****.** Histological section of an atheromatous area of the carotid plaque; dark areas represents the binding sites of Fab7816FLAG with a specific antigen present in the plaque.
**Figure 2****.** Western blotting sections showing Fab7816 binding with an antigen in the carotid lysate. Each number correspond to a distinct patient; each decimal to a distinct section of the same lesion. cubV: umbilical vein.
**Figure 3****.** Western blot assay on pathogen lysates with Fab7816. Arrows show that Fab7816 recognize with high specificity and affinity an antigen present in *Klebsiella pneumoniae* and in *Proteus mirabilis.*
**Figure 4****.** ELISA results for 7816 IgG against the bacterial antigen ompK36.
**Figure 5****.** 2D Electrophoresis (Panel A) and 2D E-WB (Panel B) assays for Fab7816 on carotid plaque lysate. Sample form spots C1 and C2 were used for the identification of the self-antigen protein.
**Figure 6****.** Western blot assays against transgelin with different antibodies. Lane TRANS: anti-TAGLN antibody; lane aMYC: anti-MYC-tag antibody conjugated with HRP; lane 78: Fab E7816Flag; lane IgG: IgG7816.
**Figure 7****.** Western blot assays against transgelin with Fab1630 and anti-TAGLN antibody. Lane "M": molecular weight standard.

### DETAILED DESCRIPTION OF THE INVENTION

The present invention refers to human antibodies characterized by the ability to bind transgelin (called also SM22) and an outer membrane bacterial protein with a high degree of homology to OmpK36 (Outer membrane protein Klebsiella 36, GI:295881594).

By transgelin the Applicant means to comprise proteins having at least 80% similarity to trangelin-1 (Accession N° Q01995 (Uniprot), GI:48255907 (NCBI). Thus, transgelin encompasses transgelin-2 (Accession N° P37802 (Uniprot), GI:12803567 (NCBI) and transgelin-3 (Accession N° Q9UI15 (Uniprot), GI:15929818 (NCBI)). Transgelin, in particular transgelin 1, has been called in the past also SM22 and is encoded by the gene TAGLN. Particularly preferred among transgelins is transgelin 1.

The antibodies herein disclosed are endowed with the further property of binding an antigen in the atherosclerotic plaque and are thus useful diagnostic reagents for atherogenic disorders.

Furthermore, since these reagents have been identified using human antibody heavy or light chain libraries prepared from atherosclerotic plaque samples as described in WO2009/037297, they are human antibodies, with the advantages deriving from being from such a source, for example in therapeutic applications. Moreover, due to their being developed within the atherosclerotic plaque they provide a hint, if not a molecular image, of the antigens involved in the *in vivo* mechanisms therein activated, representing thus unique research tools.

The terms antibody and binding agents according to the present invention are used with equivalent meaning: they refer to reagents for which a specific recognition pattern for transgelin and OmpK36 can be detected according to any of the immunologic techniques described in the following.

According to an alternative embodiment, the term refers to antibodies for which a specific binding to transgelin and to atherosclerotic plaque is detectable, either by binding to a purified, preferably recombinant, protein or fragments thereof and to an atherosclerotic plaque lysate or an immunohistology section.

According to a preferred embodiment, antibodies of the present invention are preferably selected in the group comprising at least a heavy chain variable region selected from: SEQIDNO: 2, 6, 10 and at least a light chain variable region selected from the group consisting of: SEQIDNO: 4, 8, 12 . Even more preferably they comprise the following heavy and light chain variable region combinations: SEQIDNO: 2 and SEQIDNO: 4, SEQIDNO: 6 and SEQIDNO: 8, SEQIDNO: 10 and SEQIDNO: 12. Such combinations defining functional Fabs with the immuno-specificity herein disclosed, have never been described before.

Particularly preferred Fabs are those comprising the combination of heavy chain variable region SEQIDNO: 2 and light chain variable region SEQIDNO: 4 and heavy chain 10 and light chain 12.

Additional binding reagents with the requested specificity may be obtained by expressing the following variable part of heavy and /or light chains within the proper scaffold: HC SEQ ID NO from 17 to 27 (odd numbers), coding for the aa SEQ ID NO from 18 to 28 (even numbers) and LC SEQ ID NO from 29 to 33 (odd numbers), coding for the aa SEQ ID NO from 30 to 34 (even numbers). Functional fragments of the above heavy and light chains, maintaining the binding activity for both antigens transgelin and ompK36 homologous proteins or fragments thereof, are equally comprised in the present invention. In this regard, since it is known that the most important antigen binding determining regions are complementarity determining regions (CDR) (also termed "minimal recognition units," or "hypervariable regions") and in particular CDR3 regions, particularly preferred antibodies are those comprising the following set of CDRs 1-3:

**Table 1: preferred CDRs sequences**

| code | Chain | CDR1 (SEQIDNO) | CDR2 | CDR3 |
|---|---|---|---|---|
| Fab | HC2 | GGSIGSGSYS | ISDSGNT | CARGRGILTGLFDYW |
| 7816 | | (SEQIDNO: 36) | (SEQIDNO: 38) | (SEQIDNO: 40) |
| | LC4 | QSVLDNSNHKNS | WAS | CQQYYSTPWTF |
| | | (SEQIDNO: 42) | | (SEQIDNO: 44) |
| Fab | HC6 | GGSISSSNW | IDHSGTT | CARGAKDNWGFDYW |
| 5LCx | | (SEQIDNO: 46) | (SEQIDNO: 48) | (SEQIDNO: 50) |
| | LC8 | OTI | SAT | CQHDYNDPRTF |
| | | | | (SEQIDNO: 52) |
| Fab | HC10 | GFTFSNGW | IRSNPDGGTT | CITDRGDWKWGVPRDLTYW |
| 1630 | | (SEQIDNO: 54) | (SEQIDNO: 56) | (SEQIDNO: 58) |
| | LC12 | QSVDSNY | GAY | CQQYLSPPITF |
| | | (SEQIDNO: 60) | | (SEQIDNO: 62) |
| Fab | HC24 | GFTFSDYY | ISSGGDTI | CACRGVW |
| 248 | | (SEQIDNO: 64) | (SEQIDNO: 66) | (SEQIDNO: 68) |
| | LC8 | QSISFH | GTS | COQYNWPPLTF |
| | | (SEQIDNO:70) | | (SEQIDNO: 79) |

Particularly preferred CDR sets are those corresponding to: set 1) SEQIDNO: 36, 38 and 40; set 2): SEQIDNO: 54, SEQIDNO: 56 SEQIDNO: 58, for a Heavy chain, and to set 3) SEQIDNO: 42 and 44 and set 4) to SEQIDNO: 60 and 62, for a Light chain. The above CDR sets (comprising, where present, CDRs 1, 2 and 3) may provide, once introduced in the correct scaffold, binding reagents which can be used against the antigens transgelin and OmpK36 homologous proteins or, according to a preferred embodiment, antigens in the atherosclerotic plaque.

The term "antibody" therefore further encompasses any antibody format, and includes antibodies or binding agents comprising at least one of the above mentioned HC and LC variable region combinations or functional fragments thereof, such as CDR and CDR sets able to bind specifically the antigens in any genetically engineered or synthetic realization.

Preferred antibody formats according to the invention are either Fab format or the Fc- carrying format, wherein said Fc is preferably an IgG1. In a preferred embodiment, the antibody comprises 2 sets of each preferred HC and LC combination, linked by at least one disulfide bridge. Enzymatic fragments of such preferred formats are also comprised within the present invention.

Antibody formats encompass, just by way of example, the above identified HC, LC variable region combination thereof or CDR sets 1)-4), further comprising: F(ab')2, Fab, Fab', Fv, Fc, and Fd fragments, incorporated into the following embodiments: single domain antibodies, single-chain antibodies, maxibodies, minibodies, intrabodies, diabodies, triabodies, tetrabodies, v-NAR and bis-scFv (see e.g. Hollinger and Hudson, Nature Biotechnology, 23(9):1126-1136 (2005)).

Antigen binding fragments derived from an antibody can be obtained, for example, by proteolytic hydrolysis of the antibody, for example, pepsin or papain digestion of whole antibodies according to conventional methods. By way of example, antibody fragments can be produced by enzymatic cleavage of antibodies with pepsin to provide a 5S fragment termed F(ab')2. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. Other methods for cleaving antibodies, such as separating heavy chains to form monovalent light-heavy chain fragments (Fd), further cleaving of fragments, or other enzymatic, chemical, or genetic techniques may also be used, so long as the fragments bind to the antigen that is recognized by the intact antibody.

Beside CDRs, antibody fragments may also be any synthetic or genetically engineered protein having an amino acid sequence corresponding to those herein disclosed. According to the last embodiment, the invention further discloses the polynucleotide sequences of the variable region of the Heavy and Light chains above identified and corresponding respectively to SEQIDNO:1, 5 and 9, and : SEQIDNO: 3, 7, 11. The invention encompassed vectors comprising at least one of each H and L chain variable portion combinations, in the following preferred combination of: SEQIDNO:1 and 3, SEQIDNO:5 and SEQIDNO:7, SEQIDNO:9 and SEQIDNO:11, originating the preferred combination of Heavy and Light chain Fabs. The term "antibody fragments" further includes isolated fragments consisting of the light chain variable region, "Fv" fragments consisting of the variable regions of the heavy and light chains, and recombinant single chain polypeptide molecules in which light and heavy variable regions are connected by a peptide linker (scFv proteins). Antibody fragments also comprise CDRs of the antibody. CDRs can be obtained by expressing polynucleotides encoding the CDR of interest. Preferred polynucleotide sequences in this regard, are:
SEQIDNO: 35, 37 and 39
SEQIDNO: 41 and 43
SEQ ID NO: 53, SEQ ID NO: 55 and SEQ ID NO: 57 and
SEQIDNO: 59 and 61,
encoding the CDRs of interest.

Said polynucleotides are used, for example, in the preparation of antibodies' variable regions (see, for example, Hoogenboorn H.R. et al. lmmunol Rev., 1992, 130:41-68). The binding agent may comprise at least two, three, four, five or six CDRs as described herein. The binding agent may comprise at least one variable region domain of an antibody described herein.

The variable region domains of either light and heavy chains may be further engineered by insertions, deletions, or changes in or to the amino acid sequences of the specific antibody.

In this regard, further binding reagents with the requested specificity may be obtained by expressing the following variable part of heavy and /or light chains within the proper scaffold: HC SEQ ID NO from 17 to 27 (odd numbers), coding for the aa SEQ ID NO from 18 to 28 (even numbers) and LC SEQ ID NO from 29 to 33 (odd numbers), coding for the aa SEQ ID NO from 30 to 34 (even numbers). Engineered antibodies further comprises Fab or Fab1 or functional fragments thereof, as above disclosed, covalently attached at a C-terminal amino acid to at least one other antibody domain or a fragment thereof. Thus, for example, a VH domain that is present in the variable region domain may be linked to an immunoglobulin CHI domain, or a fragment thereof. Similarly a VL domain may be linked to a CK domain or a fragment thereof. In this way, for example, the antibody may be a Fab fragment wherein the antigen binding domain contains associated VH and VL domains covalently linked at their C-termini to a CHI and CK domain, respectively. The CHI domain may be extended with further amino acids, for example, to provide a hinge region or a portion of a hinge region domain as found in a Fab' fragment, or to provide further domains, such as antibody CH2 and CH3 domains.

According to a preferred embodiment, the engineered antibody comprises preferred Fabs such as SEQIDNO 1 and 3, etc. covalently linked to a human Fc IgG1 region with sequence described in Liang, M et al. J. Immunol. Meth., 2001, 247:119-30.

The DNA encoding an antibody of the invention or fragment thereof may be propagated and expressed according to any of a variety of well-known procedures for nucleic acid excision, ligation, transformation, and transfection using any number of known expression vectors. Thus, in certain embodiments, expression of an antibody fragment may be preferred in a prokaryotic host, such as Escherichia coli (see, e.g., Pluckthun et al, Methods Enzymol, 178:497-515 (1989)). In certain other embodiments, expression of the antibody or a fragment thereof may be preferred in eukaryotic host cells, including yeasts (e.g., *Saccharomyces cerevisiae, Schizosaccharomyces pombe, and Pichia pastoris*)*,* animal cells (including mammalian cells) or plant cells. Examples of suitable animal cells include, but are not limited to, myeloma (such as a mouse NSO line), COS, CHO, or hybridoma cells. Examples of plant cells include tobacco, corn, soybean, and rice cells.

Furthermore binding agents comprise at least one of the above CDR sets and relevant nucleotide sequences cloned into known antibody framework regions (IgG1, IgG2, etc.) encoding sequences or conjugated to a suitable vehicle to enhance the half-life thereof and biological activity. Suitable vehicles include, but are not limited to Fc, polyethylene glycol (PEG), albumin, transferrin, and the like. These and other suitable vehicles are known in the art.

Engineered variants of binding agents also comprise glycosylation variants wherein the number and/or type of glycosylation site has been altered compared to the amino acid sequences of a parent polypeptide. In certain embodiments, variants comprise a greater or a lesser number of N-linked glycosylation sites than the native protein. A N- linked glycosylation site is characterized by the sequence: Asn-X-Ser or Asn-X-Thr, wherein the amino acid residue designated as X may be any amino acid residue except proline. The substitution of amino acid residues to create this sequence provides a potential new site for the addition of an N-linked carbohydrate chain. Alternatively, substitutions which eliminate this sequence will remove an existing N-linked carbohydrate chain. Also provided is a rearrangement of N-linked carbohydrate chains wherein one or more N-linked glycosylation sites (typically those that are naturally occurring) are eliminated and one or more new N- linked sites are created. Additional preferred antibody variants include cysteine variants wherein one or more cysteine residues are deleted from or substituted for another amino acid (e.g., serine) as compared to the parent amino acid sequence. Cysteine variants may be useful when antibodies must be refolded into a biologically active conformation such as after the isolation of insoluble inclusion bodies. Cysteine variants generally have fewer cysteine residues than the native protein, and typically have an even number to minimize interactions resulting from unpaired cysteines. Desired amino acid substitutions (whether conservative or non-conservative) can be determined by those skilled in the art at the time such substitutions are desired. In certain embodiments, amino acid substitutions can be used to identify important residues of antibodies to the antigens, or to increase or decrease the affinity of the antibodies to the antigens described herein. According to certain embodiments, preferred amino acid substitutions are those which: (1) reduce susceptibility to proteolysis, (2) reduce susceptibility to oxidation, (3) alter binding affinity for forming protein complexes, (4) alter binding affinities, and/or (5) confer or modify other physicochemical or functional properties on such polypeptides. According to certain embodiments, single or multiple amino acid substitutions (in certain embodiments, conservative amino acid substitutions) may be made in the naturally-occurring sequence (in certain embodiments, in the portion of the polypeptide outside the domain(s) forming intermolecular contacts). In certain embodiments, a conservative amino acid substitution typically may not substantially change the structural characteristics of the parent sequence (e.g., a replacement amino acid should not tend to break a helix that occurs in the parent sequence, or disrupt other types of secondary structure that characterize the parent sequence).

In certain embodiments, binding agents of the invention may be chemically bonded with polymers, lipids, or other moieties. Particularly preferred moieties are those comprising "contrast imaging agent" or "contrast agent", used herein interchangeably to provide for an imaging detectable moiety" or, with equivalent meaning, "imaging moiety or moieties"

These terms refer to any moiety detectable by imaging procedures, that is to say any moiety able to provide, to improve or, in any way, to advantageously modify the signal detected by an imaging diagnostic technique today in use. Among them are, for instance, magnetic resonance imaging, radio-imaging, ultrasound imaging, x-ray imaging, light imaging and the like, all of which enable the registration of diagnostically useful, preferably contrasted, images when used in association with the said techniques.

Suitable examples of the said imaging detectable moieties may thus include, for instance, chelated gamma ray or positron emitting radionuclides; paramagnetic metal ions in the form of chelated or polychelated complexes as well as of micellar systems, liposomes and microspheres; magnetic, diamagnetic or superparamagnetic coated particles, microparticles and nanoparticles; hyperpolarized NMR-active nuclei; X-ray absorbing agents including atoms of atomic number higher than 20; bubbles, microbubbles, balloons and microemulsions including biocompatible echogenic gas; reporters suitable for optical imaging including dyes, fluorescent or phosphorescent molecules, molecules absorbing in the UV spectrum, molecules capable of absorption within near or far infrared radiations, a quantum dot and, in general, all moieties which generate a detectable substance.

According to a preferred embodiment of the invention the contrast imaging agents of the invention comprise one or more binding reagents as disclosed in the present invention, with one or more imaging moieties attached to each other, either directly or through any suitable linker.

As such, just as an example, an imaging moiety could be represented by the residue of a known diagnostic agent, for instance of a chelated complex of a paramagnetic metal ion, having formula below: 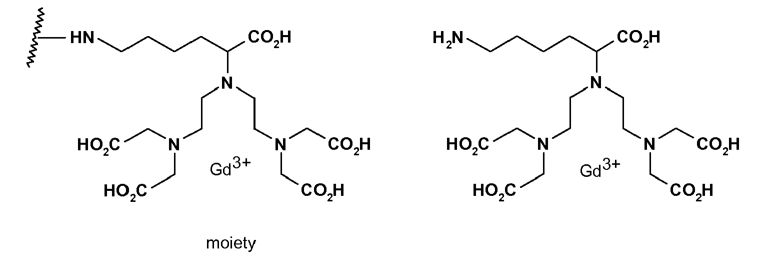 wherein the dotted line 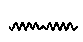 just represents the position of attachment of this moiety with the rest of the molecule.

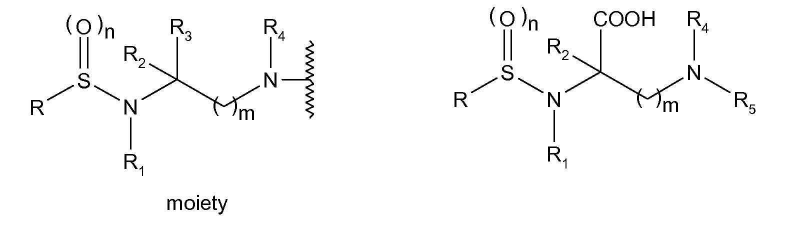

Further, and unless otherwise provided, the term "labelled with" means that the imaging moieties are attached, either directly or through suitable spacers or linkers, to the binding reagents of the present invention.

Materials detectable by diagnostic imaging modalities are known in the art, the imaging modality to be used is selected according to the imaging detectable moiety attached or linked to the binding reagent. To briefly summarize: as far as optical imaging is concerned suitable optically active imaging moieties include, for instance, optical dyes such as organic chromophores or fluorophores, having extensive delocalized ring systems and absorption or emission maxima in the range of 400-1500 nm; fluorescent molecules such as fluorescein; phosphorescent molecules; molecules absorbing in the UV spectrum; a quantum dot (e.g. fluorescent nanocrystals); or molecules capable of absorption of near or far infrared radiations.

Optical parameters to be detected in the preparation of an image may include, as an example, transmitted radiation, absorption, fluorescent or phosphorescent emission, light reflection, changes in absorbance amplitude or maxima, and elastically scattered radiation. For example, the biological tissue is relatively translucent to light in the near infrared (NIR) wavelength range of 650-1000 nm. NIR radiations can penetrate tissues up to several centimetres, permitting the use of the diagnostic agents of the invention comprising a NIR moiety to image target-containing tissues *in vivo.*

Near infrared dyes may include, for example, cyanine or indocyanine compounds such as, Cy5.5, IRDye800, indocyanine green (ICG) and derivatives thereof, including the tetrasulfonic acid substituted indocyanine green (TS-ICG), and combinations thereof.

In another embodiment, the compounds of the invention may include photolabels, such as optical dyes, including organic chromophores or fluorophores, having extensively conjugated and hence delocalized ring systems and having absorption or emission maxima in the range of 400-1500 nm. The compounds of the invention may alternatively be derivatized with bioluminescent molecules. The preferred range of absorption maxima for photolabels is between 600 and 1000 nm to minimize interference with the signal from hemoglobin. Preferably, photoabsorption labels have large molar absorptivities, e.g. > 10⁵ cm⁻¹M⁻¹, while fluorescent optical dyes have high quantum yields. Examples of optical dyes include, but are not limited to, those described in WO 96/23524.

In an embodiment of the invention, the labelling moiety for optical imaging is selected from the group of cyanine, indocyanines, phthalocyanines, naphthocyanines, porphyrins, pyrilium, azulenium or azo dyes, anthraquinones, naphthoquinones.

Preferably, within this class are fluorescein, 5-carboxyfluorescein, indocyanine green, Cy5, Cy5.5, and derivatives thereof.

The optical imaging agents described above may also be used for acousto-optical or sonoluminescent imaging performed with optically labelled imaging agents according to known methods (see, as an example: WO 98/57666). In acousto-optical imaging, ultrasound radiation is applied to the subject so as to affect the optical parameters of the transmitted, emitted or reflected light. In sonoluminescent imaging, the applied ultrasound actually generates the light detected.

As a preferred example, the above conjugation or labelling may occur between a carboxyl or amino function of the optically active imaging moiety, and the amino or carboxyl function of the binding reagents according to the invention or, optionally, with the ending amino or carboxyl functions of a linker between them.

In any case, any of the functional groups involved in the said conjugation reactions so as to give rise to the imaging agents of the invention are suitably selected in order not to reduce or modify the imaging capability of the optically active agent, nor to impair the affinity of the binding reagents of the invention.

As far as MRI contrast agents are concerned, MRI detectable moieties may comprise the residue of a chelating ligand that is labelled, in its turn, with a paramagnetic metal element detectable by MRI techniques.

Preferred paramagnetic metal elements are those having atomic number ranging between 20 and 31, 39, 42, 43, 44, 49 and between 57 and 83.

More preferred are paramagnetic metal ions selected from the following: Fe(²⁺), Fe(³⁺), Cu(²⁺), Ni(²⁺), Rh(²⁺), Co(²⁺), Cr(3⁺), Gd(³⁺), Eu(³⁺), Dy(³⁺), Tb(³⁺), Pm(³⁺), Nd(³⁺), Tm(³⁺), Ce(³⁺), Y(³⁺), Ho(³⁺), Er(³⁺), La(³⁺), Yb(³⁺), Mn(³⁺), Mn(²⁺); Gd(³⁺) being the most preferred one.

With the term "chelator", "chelating ligand" or "chelating agent", as used herein interchangeably, we intend chemical moieties, agents, compounds or molecules characterized by the presence of polar groups able to a form a complex containing more than one coordinated bond with a transition metal or another metal entity. In a preferred aspect of the invention the said chelating ligand includes cyclic or linear polyamino, polycarboxylic or polyphosphonic acids. The said ligands comprise, in addition, groups that allow for the conjugation (i.e. labelling) with the rest of the molecule. Typically, the said groups include thiol, amino or carboxyl functions either present as such or as optionally activated functions.

For MRI purposes, the chelating ligands are in their turn labelled with the selected paramagnetic metal, so as to form a chelate or coordinate complex with that metal. Suitable chelating ligands include those selected from the group consisting of: polyaminopolycarboxylic acids and derivative thereof comprising, for example, diethylenetriamine pentaacetic acid (DTPA), benzo-DTPA, dibenzo-DTPA, phenyl-DTPA, diphenyl-DTPA, benzyl-DTPA, dibenzyl DTPA, *N*,*N*-Bis[2-[(carboxymethyl)[(methylcarbamoyl)methyl]ethyl]-glycine (DTPA-BMA), N-[2-[bis(carboxymethyl)amino]-3-(4-ethoxyphenyl)propyl)]-N-[2-[bis(carboxymethyl) amino]ethyl]glycine (EOB-DTPA), 4-carboxy-5,8,11-tris(carboxymethy1)-1-phenyl-2-oxa-5,8,11-triazatridecan-13-oic acid (BOPTA), N,N-bis[2-[bis(carboxymethyl)amino]ethyl]L-glutamicacid (DTPA-GLU) and DTPA-Lys; ethylenediaminotetraacetic acid (EDTA); 1,4,7,10-teraazacyclododecane-1,4,7,-triacetic acid (DO3A) and derivatives thereof including, for example, [10-(2-hydroxypropyl)-1,4,7,10-teraazacyclododecane-1,4,7,-triacetic acid (HPD03A); 1,4,7-triazacyclononane-N,N',N"-triaceticacid (NOTA); 6-[bis(carboxymethyl)amino]tetrahydro-6-methyl-1H-1,4-diazepine-1,4(5H)-diacetic acid (AAZTA) and derivative thereof, for instance including those disclosed in WO 03/008390, 1,4,7,10-tetraazacyclotetradecane-1,4,7,10-tetraacetic acid (DOTA) and derivatives thereof including, for instance, benzo-DOTA, dibenzo-DOTA, (α,α',α",α"')-tetramethyl-1,4,7,10-tetraazacyclo-tetradecane-1,4,7,10-tetraacetic acid (DOTMA); and 1,4,8,11-tetraazacyclotetradecane-N,N',N",N"'-tetraaceticacid (TETA); or corresponding compounds wherein one or more of the carboxylic groups is replaced by a phosphonic and/or phosphinic group including, for instance, N,N'-bis-(pyridoxal-5-phosphate)-ethylenediamine-N.N'-diacetic acid (DPDP); ethylenedinitrilotetrakis(methylphosphonic)acid (EDTP), 1,4,7,10-tetraazacyclotetradecane-1,4,7,10-tetra(methylenephosphonic)acid (DOTP), the phosphonoalkyl-polyaza macrocyclic compounds disclosed in US 5,362,476 and US 5,409,689; the linear phosphonoalkyl derivatives disclosed in US 6,509,324; as well as macrocyclic chelants such as texaphirines, porphyrins and phthalocyanines.

As far as Nuclear Imaging (Radionuclide Imaging) moieties detectable by imaging techniques known in the art such as, for instance, scintigraphic imaging, Single Photon Emission Computed Tomography (SPECT) and Positron Emission Tomography (PET) may comprise the residue of a chelating agent or ligand labelled with a radionuclide detectable by the above scintigraphic, SPECT or PET imaging techniques. Suitable chelating ligands are those above reported for MRI imaging techniques and further include linear or macrocyclic ligands purposely intended for radionuclides.

Preferred metal radionuclides for scintigraphy or radiotherapy include ^{99m}Tc, ⁵¹Cr, ⁶⁷Ga, ⁶⁸Ga, ⁴⁷Sc, ⁵¹Cr, ¹⁶⁷Tm, ¹⁴¹Ce, ¹¹¹In, ¹⁶⁸Yb, ¹⁷⁵Yb, ¹⁴⁰La, ⁹⁰Y, ⁸⁸Y, ¹⁵³Sm, ¹⁶⁶Ho, ¹⁶⁵Dy, ¹⁶⁶Dy, ⁶²Cu, ⁶⁴Cu, ⁶⁷Cu, ⁹⁷Ru, ¹⁰³Ru, ¹⁸⁶Re, ¹⁸⁸Re, ²⁰³Pb, ²¹¹Bi, ²¹²Bi, ²¹³Bi, ²¹⁴Bi, ¹⁰⁵Rh, ¹⁰⁹Pd, ^{117m}Sn, ¹⁴⁹Pm, ¹⁶¹Tb, ¹⁷⁷Lu, ¹⁹⁸Au and ¹⁹⁹Au and oxides or nitrides thereof. The choice of metal will be determined based on the desired therapeutic or diagnostic application. For example, for diagnostic purposes (e.g., to diagnose and monitor therapeutic progress in primary tumors and metastases), the preferred radionuclides include ⁶⁴Cu, ⁶⁷Ga, ⁶⁸Ga, ^{99m}TC, and ¹¹¹In, with ^{99m}Tc, ¹¹¹In and ⁶⁸Ga being especially preferred.

^{99m}Tc is particularly useful and is a preferred for diagnostic radionuclide for SPECT and planar imaging because of its low cost, availability, imaging properties, and high specific activity. The nuclear and radioactive properties of ^{99m}Tc make this isotope an ideal scintigraphic imaging agent. This isotope has a single photon energy of 140 keV and a radioactive half-life of about 6 hours, and is readily available from a ⁹⁹Mo-^{99m}Tc generator. For example, the ^{99m}Tc labeled peptide can be used to diagnose and monitor therapeutic progress in primary tumors and metastases. Likewise, ⁶⁸Ga is particularly useful as it is an ideal isotope for positron emission tomography (PET). It is produced from a ⁶⁸Germanium/⁶⁸Gallium generator, thus allowing the use of a positron-emitting isotope without access to a cyclotron. Several types of ⁶⁸Ge/⁶⁸Ga generators are known to those skilled in the art. These differ in the nature of the adsorbant used to retain ⁶⁸Ge, the long-lived parent isotope, on the generator and the eluant used to elute the ⁶⁸Ga off of the column (see e.g. Fania et al, Contrast Media Mol. Imaging 2008, 3 67-77; Zhernosekov et al. J. Nucl. Med, 2007, 48, 1741-1748).

Therefore, the present invention also relates to agents for SPECT or PET imaging techniques comprising one or more residues of the binding reagents described above labelled with one or more moieties that are, in their turn, labelled with halogen radionuclides.

Means of conjugation or labelling between the binding reagents and the radioimaging detectable moiety, either directly or through a suitable linker, have been already described above for MRI agents.

According to an additional embodiment of the invention, the imaging moiety enables the formation of liposomes, microbubbles, microballoons, microspheres or emulsions and is preferably selected from the group consisting of :surfactants, sphingolipids, oligolipids, phospholipids, proteins, polypeptides, carbohydrates, synthetic or natural polymeric materials and mixtures thereof as Ultrasound contrast agents.

Preferably, the binding reagents as disclosed, comprise a residue labelled with a lipidic or phospholipidic component enabling the formation of the above liposomes, microbubbles, microballoons, microspheres or emulsions.

Interestingly, as said liposomes are formed according to conventional techniques by properly agitating these latter compounds, the liposomes thus formed will comprise, on their surface, a high number of the binding reagents according to the invention linked with the suitable moiety.

A further embodiment of the invention is thus represented by an ultrasound contrast agent in the form of liposomes, microbubbles, microballoons, microspheres or even emulsions, containing a material capable of generating an echogenic gas, further labelled with a plurality of the binding reagents according to the invention.

In the present description, and unless otherwise provided, with the term "lipid", "phospholipid" or "lipidic/phospholipidic component", as used herein, we intend a synthetic or naturally-occurring amphipatic compound which comprises a hydrophilic component and a hydrophobic component. Lipids include, for example, fatty acids, neutral fats, phosphatides, glycolipids, aliphatic alcohols and waxes, terpenes and steroids.

Examples of suitable lipids according to the invention include: phosphatidylcholines such as dioleoylphosphatidylcholine, dimyristoylphosphatidylcholine, dipalmitoyl-phosphatidylcholine and diasteroylphosphatidylcholine; phosphatidyl-ethanolamines such as dipalmitoylphosphatidylethanolamine, dioleoylphosphatidylethanolamine and N-succinil-dioleoylphosphatidyl-ethanolamine; phosphatidylserine; dipalmitoylphosphatidylserine;phosphatidylglycerols; sphingolipids; glycolipids such as ganglioside GM1; glucolipids; sulphatides; phosphatidic acid and derivatives such as dipalmitoyl phosphatidic acid (DPPA); fatty acids including palmitic, stearic, arachidonic, lauric, myristic, lauroleic, physeteric,myristoleic, palmitoleic, petroselinic, oleic, isolauric,isomyristic and isostearic fatty acids; cholesterol and derivatives such as cholesterol hemisuccinate or sulphate and cholesteryl-(4-trimethylammonio)-butanoate; polyoxyethylene fatty acids esters, alcohols or alcohol ethers; polyoxyethylated sorbitan fatty acid esters, glycerol polyethylene glycol oxy-stearate; glycerol polyethylene glycol ricinoleate; ethoxylated soybean sterols; ethoxylated castor oil; polyoxyethylene polyoxypropylene fatty acid polymers; polyoxyethylene fatty acid stearates; 1,2-dioleoyl-sn-glycerol; 1,2-dipalmitoyl-sn-3-succinylglycerol; 1,3-dipalmitoyl-2-succinylglycerol; 1-hexadecyl-2-palmitoyl-glycerophosphoethanolamine; N-succinyl-dioctadecylamine; palmitoylhomocysteine; lauryltrimethylammonium bromide; cetyltrimethyl-ammonium bromide; myristyltrimethylammonium bromide; alkyldimethylbenzylammonium chloride wherein alkyl is a C₁₂, C₁₄ or C₁₆ alkyl; benzyldimethyldodecylammonium bromide; benzyldimethyldodecyl ammonium chloride; benzyldimethylhexadecylammonium bromide; benzyldimethylhexadecylammonium chloride; benzyldimethyltetradecyl ammonium bromide; benzyldimethyltetradecyl ammonium chloride; cetyldimethylethylammonium chloride; cetylpyridinium bromide; cetylpyridinium chloride; N-[1,2,3-dioleoyloxy)-propyl]-N,N,N-trimethylammonium chloride (DOTMA); 1,2-dioleoyloxy-3-(trimethylammonium)propane (DOTAP); and 1,2-dioleoyl-c-(4'- trimethylammonium)-butanoyl-sn-glycerol (DOTB).

With the term "liposomes", as used herein, we refer to a generally spherical cluster or aggregate of amphipathic compounds, including lipid/phospholipid compounds, typically in the form of one or more concentric layers, for example bilayers. They may also be referred to herein as lipid vesicles.

With the term "vesicle", as used herein, we refer to a spherical entity which is characterized by the presence of an internal void. Preferred vesicles are formulated from lipids, including the various lipids described herein and, in any given vesicle, the lipids may be in the form of monolayer or bilayer.

The lipid vesicles described herein include such entities commonly referred to as liposomes, micelles, bubbles, microbubbles, microspheres and the like. The internal void of the vesicles may be filled with a gas or a gaseous precursor.

The term "bubbles", as used herein, refers to a vesicle which is generally characterized by the presence of one or more membranes or walls surrounding an internal void that is filled with a gas or a gas precursor.

The terms "microspheres" and "microballoons", as used herein, preferably refer to spheres having a diameter of less than, or equal to, 10 microns.

These microballoons have an envelope including a biodegradable physiologically compatible polymer or a biodegradable solid lipid. The polymers useful for the preparation of the microballoons of the present invention can be selected from the biodegradable physiologically compatible polymers such as any of those described in: EP 458745.

Polymer can be selected from biodegradable physiologically compatible polymers, such as polysaccharides of low water solubility, polylactides and polyglycolides and their copolymers, copolymers of lactides and lactones such as ε-caprolactone, γ-valerolactone and polypeptides.

The microballoons of the present invention can also be prepared according to the methods disclosed in WO 96/15815, where the microballoons are made from a biodegradable membrane comprising biodegradable lipids, preferably selected from mono- di-, tri-glycerides, fatty acids, sterols, waxes and mixtures thereof. Preferred lipids are di- or tri-glycerides, e.g. di- or tri-myristin, -palmityn or -stearin, in particular tripalmitin or tristearin.

The microbaloons may employ any of the gases disclosed herein or known to the skilled artisan for ultrasound techniques.

Any biocompatible gas may be used in the vesicular contrast agents of the invention. The term "gas", as used herein, includes any substance (comprehensive of mixtures thereof) substantially in gaseous form at the normal human body temperature.

Said gas may thus include, for example, air, nitrogen, oxygen, CO₂, argon, xenon, krypton, fluorinated gases (including, for example, perfluorocarbons, SF₆ or SeF₆) and low molecular weight hydrocarbons (for instance those containing from 1 to 7 carbon atoms including alkanes such as methane, ethane, propane, butane or pentane; cycloalkanes such as cyclopropane, cyclobutane or cyclopentane; alkenes or alkynes such as ethylene, propene, propadiene, butene, acetylene, propyne, and/or mixtures thereof). Fluorinated gases are however preferred. Fluorinated gases include materials which contain at least one fluorine atom. Examples include, but are not limited to, compounds such as SF₆, freons (organic compounds containing one or more carbon atoms and fluorine such as CF₄, C₂F₆, C₃F₈, C₄F₈, C₄F₁₀, CBrF₃, CCl₂F₂, C₂CIF₅ and CBrCIF₂) and perfluorocarbons.The term "perfluorocarbon" refers to compounds containing only carbon and fluorine atoms and include saturated, unsaturated and cyclic perfluorocarbons.

The saturated perfluorocarbons, which are preferred, have the formula CₙFₙ₊₂, where n is from 1 to 12, preferably from 2 to 10, more preferably from 3 to 8 and even more preferably from 3 to 6. Suitable perfluorocarbons thus include, but are not limited to, CF₄, C₂F₆, C₃F₈, C₄F₈, C₄F₁₀, C₅F₁₂, C₆F₁₂, C₇F₁₄, C₈F₁₈ and C₉F₂₀. More preferably, the gas or gas mixture comprises SF₆ or a perfluorocarbon selected from the group consisting of: C₃F₈, C₄F₈, C₄F₁₀, C₅F₁₂, C₆F₁₂, C₇F₁₄, C₈F₁₈, with C₄F₁₀ being particularly preferred.

As an additional embodiment of the invention the above liposomes, micellar systems, vesicles, microspheres or microballoons, may entrap other imaging moieties among those previously disclosed as further macromolecular aggregates embodiment for diagnostic imaging.

We thus refer, according to an additional embodiment of the invention, to a macromolecular system for use in MRI imaging techniques comprising the above liposomes, micellar systems, vesicles, microspheres or microballoons, being prepared according to conventional methods by starting from the compounds comprising a binding reagent as above disclosed, properly labelled with a lipidic or phospholipidic component as set forth above, and wherein within the cavity of the said liposomes, micellar systems, vesicles, microspheres or microballoons, there are incorporated suitably chelated MRI paramagnetic metal ions.

Preferably, an additional object of the invention is thus represented by the liposomes obtained labelling the binding reagents with lipidic or phospholipidic components, and wherein the inner cavity of the said liposomes comprises the aforementioned chelate complexes of Gd³⁺ ions or chelated ligands of lanthanide ions for MRI purposes.

Said liposomial imaging agents are characterized by an enhanced sensitivity over traditional MRI contrast agents as they may take advantage of the difference in NMR (Nuclear Magnetic Resonance) signal intensity of water protons in the presence and in the absence of the contrast agent, when a radiofrequency is applied, the said radiofrequency corresponding to the resonance frequence of water protons exchangeable by the system, that is inside and outside the liposomial vesicle.

Such contrast amplification technique is better known as Chemical Exchange Saturation Transfer (CEST) and the materials suitable for the said technology are better known as LIPOCEST, hence of chelated complexes of lanthanide ions entrapped within liposomial vescicles that, according to the present invention, are labelled with a plurality of the binding reagents according to the invention.

In the present invention, the CEST imaging system is represented by a liposomal system. In this case, the chemical shift of the intraliposomal water protons which must be irradiated to observe saturation transfer has been suitably "shifted" as a result of their interaction with a paramagnetic chelate containing a lanthanide metal ion.

The paramagnetic complex can be encapsulated in the aqueous cavity of the liposome (if hydrophilic), and/or incorporated in the lipidic bilayer of the membrane (if amphiphilic).

For a general reference to CEST techniques and LIPOCEST(s) see, as an example, Angew. Chem. Int. Ed. 2007, 46, 966-968; and Chem. Commun., 2008, 600-602.

All the above variants and diagnostic derivatives are deemed to be comprised in the present invention, provided that their affinity to the antigens transgelin and OmpK36 homologous proteins is not significantly altered, with respect to the preferred binding reagent embodiment.

Immunoaffinity to (transgelin 1 Accession Q01995, G148255907) is usually maintained for protein having at least 80%, more preferably at least 82%, 84%, 86%, 88%, 90%, 92%, 94%, 96%, 98%, 99% sequence similarity with the human transgelin 1 sequence.

The same is true for antibodies binding OmpK36 (Klebsiella OmpK36 GI:295881594) even though with a lower degree of homology (based on amino acid similarity) with the OmpK36 protein itself, maintained for protein having at least 50% preferably at least 60%, 70%, 80% 82%, 84%, 86%, 88%, 90%, 92%, 94%, 96%, 98%, 99% sequence similarity to the OmpK36 sequence; where for sequence similarity intermediate values are also comprised within the scope of the invention.

Antibodies which are particularly preferred are those able to immunologically recognize transgelin and the outer membrane protein of either *Proteus vulgaris* or *Klebsiella,* even more preferably transgelin 1 and Klebsiella OmpK36, by immunoassay, whether performed at the same time or different or in a sequential order.

According to an alternative embodiment the antigens recognized by the present antibodies, are transgelin fragments, preferably obtained by synthesis, selected in the group consisting of: N-terminal fragments, comprising aa 5-18, even more preferably aa 3-20 or even more preferably aa 1-30; C-terminal fragments, comprising aa 185-198, even more preferably comprising aa 170-199, or even more preferably comprising aa 160-201 according to the numbering of the sequence in the reference database.

For OmpK homologous proteins, an embodiment alternative to the use of the whole protein, either native or recombinant, is the use of peptides selected in the group consisting of: the OmpK N-terminal region comprising aa 1-20; a fragment comprising aa 70-80, or even more preferably comprising aa 68-90; a fragment comprising aa 130-155, or even more preferably comprising aa 125-160; a fragment comprising aa 250-290, preferably comprising aa 263-276. These peptides from both transgelin and OmpK 36, are reagents useful for screening antibodies according to the present invention and are therefore also comprised within its scope.

Beside the above identified isolated antigens or fragments thereof, even more preferably, the binding reagents of the present invention bind *in vitro* biopsies of the atherosclerotic plaque, more preferably taken from a coronary plaque, as it can be shown by immunoassays on plaque tissue lysate, i.e. by immunoprecipitation or western blot, or by immunohistology on plaque sections.

Without being bound to a particular hypothesis, it is plausible that antibodies against an antigen sharing epitope(s) with either transgelin or Outer membrane proteins homologous to the OmpK36 from Klebsiella, develop as an immune or autoimmune response triggered by inflammation within the vessels, or in other words, as a response to the well characterized atherogenic mechanisms. Therefore according to a further embodiment, the present invention relates to a method for preparing anti-idiotipic antibodies, where the binding reagents herein disclosed are used as a molecular image of the "atherogenic" antigen. The preparation of anti-idiotipic antibodies is well known to the skilled person and is described for example in Burioni R, et al. PLoS One. 2008;3(10):e3423. Anti-idiotipic antibodies may represent a vaccine protecting from the development of atherosclerosis.

The present invention further relates to a method for the *in vitro* or *in vivo* immunodetection of a sample from an atherosclerotic plaque, where the antibodies according to the present invention are used. Immunoassays take a variety of forms which are all comprised within the scope of the present invention. Conventional assays may be carried out by ELISA, Western blot, immunoprecipitation, immunofluorescence, immunochemistry or FACS. Further, immunoassays according to the invention may be fluorescent immunoassays, chemiluminescent assays, agglutination assays, nephelometric assays, turbidimetric assays, Western Blots, competitive or non-competitive immunoassay, homogenous or heterogenous immunoassays, and reporter-assays, e.g. a luciferase assay. Reference may be made to any manual such as: "Current Protocols in Immunology", 1994 ed.

According to a preferred embodiment, the immunoassay is an ELISA or a Western-blot where atherosclerotic plaque lysate is used as a preferred capture antigen.

Furthermore and according to a preferred embodiment, the immunoassay provides for the detection in a sample, of antibodies binding the antigens: transgelin or fragment, variant or derivative thereof and proteins homologous to OmpK36, either at the same time or in succession, preferably by using the antibody of the present invention as competitive binding agents, where the presence of antibodies in an unknown biological sample, i.e. a patient's serum, preferably competing with those according to the present invention, are indicative that an atherogenic process has been triggered and/or is developing or that an atheromatous disease is in course. These antibodies may optionally further bind to histological sections of atherosclerotic plaques in or developing in arterial vessels, namely coronary or carotid.

Even though these assays may be realized with several technical variants comprised within the scope of the present invention, one of the preferred embodiment is by competitive assays, where at least one of the antibodies according to the present invention is used as a competing reagent with the antibodies in the biological sample in binding to transgelin and OmpK36.

An example of immunoassay realization comprises contacting the antigens with an antibody or fragment or derivative thereof according to the invention and detecting the level of a complex comprising said antibody and transgelin and between said antibody and proteins homologous to OmpK36, variant or derivative thereof in the presence or absence of the sample.

As discussed above, any suitable technique for determining formation of the complex or, by reverse, inhibition of complex formation may be used.

Competitive immunoassays include but are not limited to: radioimmunoassays (RIAs), enzyme-linked immunosorbent assays (ELISAs) and immunochromatographic techniques (ICTs), Western blotting which are well known to those skilled in the art. It will be understood that the present invention encompasses qualitative and quantitative immunoassays.

Suitable immunoassay techniques include both single-site and two-site assays of the non-competitive types, as well as the traditional competitive binding assays. These assays also include direct binding of a labelled antigen-binding molecule to a target antigen wherein said target antigen is transgelin or fragments thereof and proteins homologous to OmpK36 or fragments thereof.

Two-site assays are particularly favoured for use in the present invention. A number of variations of these assays exist, all of which are intended to be encompassed by the present invention. Briefly, in a typical forward assay, an unlabelled antigen-binding molecule such as an unlabelled antibody is immobilized on a solid substrate and the sample to be tested brought into contact with the bound molecule. After a suitable period of incubation, for a period of time sufficient to allow formation of an antibody-antigen complex, another antigen-binding molecule, suitably a second antibody specific to the antigen, labelled with a reporter molecule capable of producing a detectable signal is then added and incubated, allowing time sufficient for the formation of another complex of antibody-antigen-labelled antibody. Any unreacted material is washed away and the presence of the antigen is determined by measuring or detecting a signal produced by the reporter molecule. The results may be either qualitative, by simple observation of the visible signal, or may be quantitated by comparing with a control sample containing known amounts of antigen. Variations on the forward assay include a simultaneous assay, in which both sample and labelled antibody are added simultaneously to the bound antibody. These techniques are well known to those skilled in the art, including minor variations as will be readily apparent.

For quantitative data, and as a general practice, positive and negative controls may be used, as known by the skilled man. Positive standards may comprise the human recombinant protein transgelin (either flagged or not) and antibodies specific against it (or the flag), which are both commercially available. OmpK36 from Klebsiella can be easily produced by standard recombinant techniques, starting from the nucleotide sequence encoding for it.

In the typical forward assay, a first antibody having specificity for the antigen or antigenic parts thereof is either covalently or passively bound to a solid surface. The solid surface is typically glass or a polymer, the most commonly used polymers being cellulose, polyacrylamide, nylon, polystyrene, polyvinyl chloride or polypropylene. The solid supports-may be in the form of tubes, beads, discs of microplates, or any other surface suitable for conducting an immunoassay. The binding processes are well known in the art and generally consist of cross-linking covalently binding or physically adsorbing, the polymer-antibody complex is washed in preparation for the test sample. An aliquot of the sample to be tested is then added to the solid phase complex and incubated for a period of time sufficient and under suitable conditions to allow binding of any antigen present to the antibody. Following the incubation period, the antigen-antibody complex is washed and dried and incubated with a second antibody specific for a portion of the antigen. The second antibody has generally a reporter molecule associated therewith that is used to indicate the binding of the second antibody to the antigen. The amount of labelled antibody that binds, as determined by the associated reporter molecule, is proportional to the amount of antigen bound to the immobilized first antibody.

An alternative method involves immobilizing the antigen in the biological sample and then exposing the immobilized antigen to specific antibody that may or may not be labelled with a reporter molecule. Depending on the amount of target and the strength of the reporter molecule signal, a bound antigen may be detectable by direct labelling with the antibody. Alternatively, a second labelled antibody, specific to the first antibody is exposed to the target-first antibody complex to form a target-first antibody-second antibody tertiary complex.

The complex is detected by the signal emitted by a reporter molecule. Suitable reporter molecule associated with the antigen-binding molecule may include the following:- (a) direct attachment of the reporter molecule to the antibody; (b) indirect attachment of the reporter molecule to the antibody; i. e. , attachment of the reporter molecule to another assay reagent which subsequently binds to the antibody; and (c) attachment to a subsequent reaction product of the antibody. The reporter molecule may be selected from a group including a chromogen, a catalyst, an enzyme, a fluorochrome, a chemiluminescent molecule, a paramagnetic ion, a lanthanide ion such as Europium (Eu), a radioisotope including other nuclear tags and a direct visual label.

In the case of a direct visual label, use may be made of a colloidal metallic or non-metallic particle, a dye particle, an enzyme or a substrate, an organic polymer, a latex particle, a liposome, or other vesicle containing a signal producing substance and the like.

A large number of enzymes suitable for use as reporter molecules include alkaline phosphatase, horseradish peroxidase, luciferase, P-galactosidase, glucose oxidase, lysozyme, malate dehydrogenase and the like. The enzymes may be used alone or in combination with a second enzyme that is in solution.

Suitable fluorochromes include, but are not limited to, fluorescein isothiocyanate (FITC), tetramethylrhodamine isothiocyanate (TRITC), R-Phycoerythrin (RPE), and Texas Red. Other exemplary fluorochromes include those discussed by Dower et al.: WO 93/06121.

In the case of an enzyme immunoassay, an enzyme is conjugated to the second antibody, generally by means of glutaraldehyde or periodate. As will be readily recognized, however, a wide variety of different conjugation techniques exist which are readily available to the skilled artisan. The substrates to be used with the specific enzymes are generally chosen for the production of, upon hydrolysis by the corresponding enzyme, a detectable colour change. Examples of suitable enzymes include those described supra. It is also possible to employ fluorogenic substrates, which yield a fluorescent product rather than the chromogenic substrates noted above. In all cases, the enzyme-labelled antibody is added to the first antibody-antigen complex, allowed to bind, and then the excess reagent washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample.

Alternately, fluorescent compounds, such as fluorescein, rhodamine and the lanthanide, europium may be chemically coupled to antibodies without altering their binding capacity. When activated by illumination with light of a particular wavelength, the fluorochrome-labelled antibody adsorbs the light energy, inducing an excited state in the molecule, followed by emission of the light at a characteristic colour visually detectable with a light microscope. The fluorescent-labelled antibody is allowed to bind to the first antibody-antigen complex. After washing off the unbound reagent, the remaining tertiary complex is then exposed to light of an appropriate wavelength. The fluorescence observed indicates the presence of the antigen of interest. Immunofluorometric assays (IFMA) are well established in the art and are particularly useful for the present method.

For solid phase assays, suitable solid support materials are nitrocellulose, polyvinylchloride or polystyrene, e.g. the well of a microtiter plate.

The invention also provides a diagnostic kit comprising at least one anti-transgelin and OmpK36 binding reagent according to the present invention. The binding reagent is preferably a Fab-IgG1 as disclosed above. In addition, such a kit may optionally comprise one or more of the following:
(1) instructions for using the one or more binding agent(s) for screening, diagnosis, prognosis, therapeutic monitoring or any combination of these applications;
(2) a labeled binding partner to the binding agent(s) of the invention; (3) a solid phase (such as a reagent strip) upon which the binding agent(s) is immobilized; and
(4) a label or insert indicating regulatory approval for screening, diagnostic, prognostic or therapeutic use or any combination thereof.

If no labeled binding partner to the binding agent(s) is provided, the binding agent(s) itself can be labeled with one or more of a detectable marker(s), e.g., a chemiluminescent, enzymatic, fluorescent, or radioactive moiety.

According to a further aspect, the invention provides for pharmaceutical compositions, as injectable formulations for diagnostic purposes, comprising one of the above-described binding reagents along with a pharmaceutically or physiologically acceptable carrier, excipient, or diluent. The development of suitable dosing and treatment regimens for using the particular compositions described herein in a variety of treatment regimens, including e.g., subcutaneous, oral, parenteral, intravenous, intranasal, and intramuscular administration and formulation, is well known in the art, some of which are briefly discussed below for general purposes of illustration. In certain circumstances it will be desirable to deliver the pharmaceutical compositions disclosed herein subcutaneously, parenterally, intravenously, intramuscularly, or intra-peritoneally. In certain embodiments, solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations generally will contain a preservative to prevent the growth of microorganisms. Illustrative pharmaceutical forms suitable for injectable use include sterile aqueous solutions or dispersions and sterile powders for the extemporaneous preparation of sterile injectable solutions or dispersions (for example, see U.S. Patent No. 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, liquid polyethylene glycol, and the like), suitable mixtures thereof, and/or vegetable oils. Proper fluidity may be maintained, for example, by the use of a coating, such as lecithin, by the maintenance of the required particle size in the case of dispersion, and/or by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Moreover, for human administration, preparations will preferably meet sterility, pyrogenicity, and the general safety and purity standards as required by FDA Office of Biologics standards.

In another embodiment of the invention, the compositions disclosed herein may be formulated in a neutral or salt form. Illustrative pharmaceutically- acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine, and the like. Upon formulation, solutions will be administered in a manner compatible with the dosage formulation and in such amount as is therapeutically effective.

The carriers can further comprise any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase "pharmaceutically-acceptable" refers to molecular entities and compositions that do not produce an allergic or similar untoward reaction when administered to a human.

In certain embodiments, liposomes, nanocapsules, microparticles, lipid particles, vesicles, and the like, are used for the introduction of the compositions of the present invention into suitable host cells/organisms. In particular, the compositions of the present invention may be formulated for delivery either encapsulated in a lipid particle, a liposome, a vesicle, a nanosphere, or a nanoparticle or the like. Alternatively, compositions of the present invention can be bound, either covalently or non-covalently, to the surface of such carrier vehicles. The formation and use of liposome and liposome-like preparations as potential drug carriers is generally known to those of skill in the art (see for example, Lasic, Trends Biotechnol, 16(7):307-21 (1998); Takakura, Nippon Rinsho, 56(3):691-95 (1998); The use of liposomes does not appear to be associated with autoimmune responses or unacceptable toxicity after systemic delivery. In certain embodiments, liposomes are formed from phospholipids that are dispersed in an aqueous medium and spontaneously form multilamellar concentric bilayer vesicles (also termed multilamellar vesicles (MLVs)).

According to further aspects the invention provides for the use of the binding reagents of the invention as diagnostic tools in the early detection of atherogenic diseases wherein said term comprises an atherogenic ischemic or occlusive evolution in an arterial vessel, further comprising Acute Coronary syndrome and related cardiovascular diseases such as unstable angina, ST Elevation Myocardial Infarction (STEMI), nonSTEMI myocardial infarction) as well as intra-cerebral occlusive disease (STROKE) or peripheral artery occlusive diseases....

In summary, the invention provides for the following embodiments covering detection methods, preferably immuno-detection methods, even more preferably Western-blot and ELISA assay either:
a) for the detection of antibodies against an antigen/(epitope) in an atherosclerotic plaque, said method comprising allowing an unknown biological sample to react with human transgelin-1 or fragments thereof, optionally in competition with an antibody comprising at least a heavy chain selected from: SEQIDNO: 2, 6, 10, 14 and at least a light chain selected from the group consisting of: SEQIDNO: 4, 8, 12, 16. According to a preferred embodiment, the method comprises using the following heavy and light chain combinations: SEQIDNO: 2 and SEQIDNO: 4, SEQIDNO: 6 and SEQIDNO: 8, SEQIDNO: 10 and SEQIDNO: 12, SEQIDNO: 14 and SEQIDNO: 16, as competing antibodies.

The method further comprises allowing said unknown biological sample to react with a protein homologous to OmpK36 or fragments thereof, as described above. The method further provides to define as positive the biological sample where a specific binding to both antigens can be detected.
b) for the detection of an antigen/(epitope) in the atherosclerotic plaque of an unknown sample comprising allowing said unknown biological sample to react with an antibody comprising at least a heavy chain selected from: SEQIDNO: 2, 6, 10, 14 and at least a light chain selected from the group consisting of: SEQIDNO: 4, 8, 12, 16.

The method further provides to define as positive the biological sample where a specific binding to a region of the atherosclerotic plaque can be detected. Immunodetection methods based on the binding reagents herein disclosed allows the detection of an atherogenic process or ateromatous diseases, wherein said term comprises an atherogenic ischemic or occlusive evolution in an arterial vessel, further comprises Acute Coronary syndrome comprising unstable angina, ST Elevation Myocardial Infarction (STEMI), nonSTEMI myocardial infarction) and related cardiovascular diseases, as well as intra-cerebral occlusive disease (i.e. stroke), peripheral artery occlusive diseases or non-acute coronary diseases. Furthermore, the invention comprises transgelin, preferably transgelin-1, as a marker of atherogenesis and its use as a marker of atherogenesis or of the presence of an atherosclerotic plaque in immunodetection methods, which according to a preferred embodiment, further comprise the detection of such a binding specificity in combination with the detection of a binding specificity to a bacterial outer membrane protein selected among those with at least 50% similarity to OmpK36, as defined above.

The following experimental examples are offered by way of illustration, and not by way of limitation.

### EXPERIMENTAL PART

### Example 1: Preparation of Mab minilibrary form the coronary plaque

The preparation of cDNA of Fab libraries from atherosclerotic plaques has been described in W02009/037297.

Briefly: a sufficient amount of tissue (usually about 1-2 mg) was obtained from the atherosclerotic plaque from a number of patients with acute coronary syndrome and undergoing coronary atherectomy, and stored in liquid nitrogen, with the patient's informed consent.

The tissue was homogenized and the total mRNA was extracted according to conventional methodologies using a commercial kit for the extraction of mRNA. Reverse transcription of mRNA was performed using a commercial kit for the retrotranscription of mRNA. The cDNA synthesis is performed according to standard procedures from the total mRNA primed with oligo(dT).

1 µl of cDNA was used for polymerase chain reaction. Reverse primers were designed in order to anneal to the segments of sequences coding for the constant region of heavy and light chains respectively. The PCR products of heavy and light chains of a Fab (variable region and the CH1 domain) amplified from the human biopsy, were cloned into a phagemidic vector (pRB32) to allow the combinatorial generation or heavy and light chain pairs exposed (phage display) onto the external phage surface the Fab fragment codified by the DNA cloned into the phagemid. This was obtained by cloning in frame the heavy chain fragment with a phage M13 membrane protein.

This allowed the generation of a combinatorial antibody Fab fragment phage-display libraries which have been screened with the lysates and antigens described in the following examples

### Example 2: Phage display libraries selection by biopanning on cell, carotid or bacterial lysates.

Hep-2 (ATCC n° CCL-23) cell lysates were prepared growing the cells in E-Mem (Invitrogen 0820234DJ) supplemented with antibiotic/antimycotic Solution (Invitrogen, Antibiotic/Antimycotic Solution, liquid 15240-062) and 10% FBS. Cells were regularly split 1:10 every five days. Five million cells were washed in PBS al lysed by using RIPA buffer (50mM Tris HCL pH8 + 150 mM NaCl + 1% NP-40 + 0.5% NA deossicolate + 0.1 % SDS).

Carotid lysates were prepared from a portion (10 g) of human atherosclerotic carotid plaque obtained as described above. Carotid plaques were immersed in RIPA buffer and homogenized with Tissue ruptor (Qiagen).

Bacterial cell lysates were carried out inoculating a colony or a scratch in 10 ml of SB and growing bacteria at 37°C overnight. Cultures of *Stapylococcus aureus, Proteus mirabilis, Klebsiella pneumoniae, Enterococcus cloacae, Streptococcus pyogenes, Neisseriae gonhorreae, Listeria monocytogenes* were grown. Overnight cultures were harvested by centrifugation (4500 RPM for 10 min.) and then resuspended in 1 ml of RIPA buffer and protease inhibitor PMSF (1mM) final). After three cycles of freeze-and-thawing (37°C and -80°C) and one cycle of sonication (90 sec at max power) the lysate was cleared by centrifugation (15000 RPM at 4°C for 20 min). Moreover one colony of *Haemophilus influenzae* was seeded on blood agar plates and then grown at 37°C overnight. Haemophilus inluenzae colonies were collected and resuspended in 500 µl of RIPA/PMSF. All lysates were stored at -20°C until usage.

Bacterial lysates were used to perform immunoaffinity selection by biopanning with the Fab libraries described in example 1 or as described for example in a manual Phage Display. A laboratory manual, C. Barbas, D. Burton, J. Scott, G. Silvermann Jan. The night before biopanning, an ELISA plate was coated O/N at 4°C with 50 microL/well of antigen(s) (100 ng/well) solution in appropriate coating buffer. After washing with deionised H₂O, wells were blocked completely with PBS/BSA 3%, sealed and incubated in a humidified container for 1 h at 37°C. Fifty microL phage suspension was added to each well (total of about 10¹¹ PFU) and incubated for at least 2 h at 37°C after sealing the plate. Phage were removed from every well (and kept for titration), washed 10 times with PBS/Tween 0.05%. After intensive washing, phage bound to the antigen were eluted by washing each well with 50 microL of Elution Buffer (0.1 M HCl, adjusted with glycine to pH=2.2, BSA 1 mg/ml) and adding to the eluent 3 µl of 2M Tris base. After the elution, 2 ml of fresh XL-1 blue *E.coli* (Stratagene) were infected with the eluted phage. After an incubation at 37°C for 15 min, 10 ml of 37°C-prewarmed Superbroth (SB) (20 µg/ml ampicillin and 10 µg/ml tetracycline) were added. After incubation for 1 hat 37°C on a shaker, 100 ml of SB containing 100 µg/ml Amp and 10 microG/ml Tet were added to each 10 ml-culture and incubated for 1 h at 37° C on a shaker. A helper phage VCS M13 (total of 10¹² PFU) was used to infect the culture and incubated on the shaker for 2 h at 37°C. After addition of kanamycin (70 µg/ml) cultures were incubated on a shaker O/N at 30 °C.

The day after, cells were spun down at 6000 RPM (Sorvall SS34), 4°C for 15 min. and PEG8000 (Sigma-Aldrich) was added to the supernatant to a final volume of 20ml. Phages were precipitated on ice for 30 min. and then centrifuged at 11,000 RPM (Sorvall SS34) for 20 min. at 4 °C). Phages where then resuspend in 2 ml PBS/BSA 1 %, and stored at 4 °C for subsequent biopanning rounds.

To verify the best coating conditions one microgram of purified ompK36 was diluted in different buffers (PBS, Carbonate/Bicarbonate or NaHCO₃) and the fraction coated on the plate was estimate by ELISA assay using anti-HIS-Peroxidase antibody.

The optimal conditions for coating were pH 5.0 with PBS buffer, overnight at 4°. Lysates and Omp were coated in PBS (pH 5.2)

After each elution and round of selection, eluted phage were titered in order to evaluate the efficacy of the enrichment procedure. The procedure was repeated four times allowing enrichment of selected populations.

### Example 3: Sequencing

Clones obtained according to the biopanning procedure carried out as described in Example 2 and 13 for libraries derived from different coronary plaques, were sequenced for quantitative and qualitative analysis and sequencing by Big Dye chemistry and analyzed using ABI PRISM 3100 sequencer.

Screening of the minilibrary from a coronary plaque (IDN° 11: ID11LIB) provided for a heavy chain corresponding to SEQ ID NO: 1 and coding for the amino acidic sequence corresponding to SEQ ID NO: 2. The light chain correspond to SEQ ID NO: 3 and coding for the amino acidic sequence corresponding to SEQ ID NO: 4.

### Example 4. Expression system for SEQINO 1 and 3

Clone of the heavy chain (corresponding to SEQ ID NO: 1) and clone of the light chain (corresponding to SEQ ID NO: 3) of the coronary plaque sample were selected for recombinant expression of the soluble Fab fragments according to the procedure described in Burioni R. et al. J. Immunol. Methods, 1998, 217(1-2):195-9.

Two expression systems were used: the first one allowing the production of flagged Fabs for purification (ref above) the second for secondary recognition (Burioni R. et al. Hum. Antibodies, 2001, 10(3-4):149-54).

Purified Fabs were tested in SDS-PAGE gel in non-reducing conditions showing a single band of approximately 50 kDa.

### Example 5. Characterization of the recombinant Fabs by immunohistology.

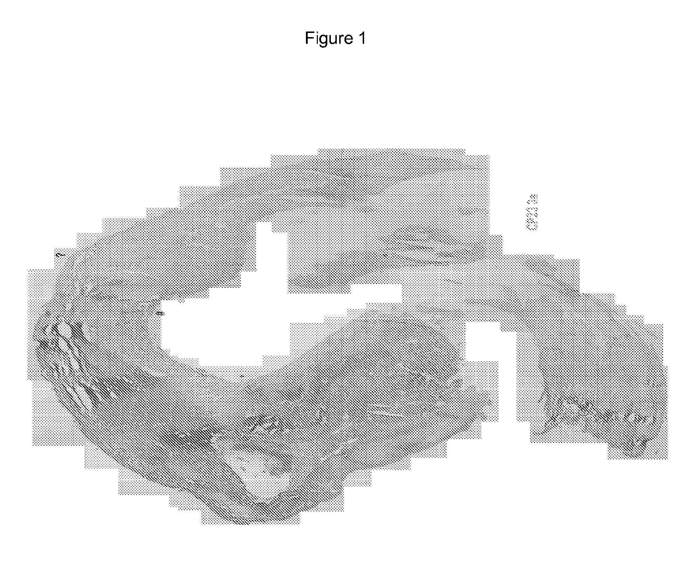

Carotid plaques harvested during TEA surgery, just after excision were fixed in 4% paraformaldehyde in PBS, cut in consecutive 2mm thick rings, decalcified in EDTA solution when required, cryoprotected in 10% sucrose in PBS, embedded in OCT compound, then snap frozen in isopentane/liquid nitrogen and stored at -80°C. Cryosections (5 mm thick) were cut from every ring and stained with either Hematoxylin/Eosin or Movat's pentachrome, thus the morphology was examined in at least 4 sections/ring using an Eclipse 55i microscope equipped with DS-L1 camera and LUCIA 4.82 software (all from Nikon, Tokyo, J). Multiple images collected from each ring, were composed by LUCIA 4.82 software, to obtain a whole section picture. The presence of thin cap, foam cells, fibrolipids, ulcerations, necrotic/thrombotic core, calcifications, hemorrhage/thrombosis and inflammatory infiltrate were used to classify carotid plaques as stable, vulnerable, or unstable, following Virmani's modified AHA classification. Additional sections (10µm thick) from rings with and without atheroma were selected and submitted for immunolabeling with anti CD138 antibody or with 7816Fab-FLAG detected with anti-FLAG clone M2 followed by rabbit-anti-Mouse IgG AlexaFluor488. DAPI stained the nuclei. Sections were examined using a Leica TCS SP2 confocal microscope (Leica Microsystems, Heidelberg, GmbH); 2D projection were obtained from Z-series of images and superposed by Adobe PhotoshopCS software. A histological immuno-stained section is shown in Figure 1.

### Example 6: Characterization of the recombinant Fabs by Western Blotting on carotid lysates

Carotid plaques were macrodissected in 2mm-thick rings and frozen in isopentane/ liquid nitrogen. Every ring was considered as a single specimen. For each patient proteins were extracted from a stenotic and a non-stenotic ring by tissue homogenization in RIPA lysis buffer. The protein content of carotid plaque homogenates was quantified by Bradford assay. Western blot was performed on protein extracts (20 µg/lane) by using 10% Sodium Dodecyl Sulphate - PolyAcrylamide Gel Electrophoresis (SDS-PAGE). SDS-PAGE loading buffer was added to protein lysates before heating at 90°C for 5 min. Gel was run for about 2 hours at 120V. PVDF membrane was treated according to the manufacturer's instruction and proteins were transferred to membrane at 0,350 mA for 2 hours. The membrane was stained by Ponceau red to verify the proper transfer. Then the membrane was blocked with a solution of PBS1x/BSA10% at 4°C in agitation overnight. The day after the membrane was incubated with Fab E7816 (10 µg/ml) diluted in PBS1x/BSA5% for 1 hour at R.T. and then was washed three times with PBS1x/Tween-20 0,1 %. Target (7816-FLAG) and reference proteins (bActin) were detected with specific antibodies (anti-FLAG clone M2 followed by Rabbit-anti Mouse IgG-HRP, and Rabbit-anti Mouse IgG-HRP, respectively - all from Sigma (cod: A9917, St. Louis, MO). The membrane was developed by SuperSignal West Pico Chemiluminescent Substrate (Pierce) according to the manufacture instructions. The mean pixel optical density (OD) for each band of the target protein, determined by densitometry, was normalized vs. β-Actin, to evaluate its quantity with respect to carotid plaque total cells. Stenotic and non stenotic rings from stable and unstable (determined by US) carotid plaque specimens were compared.

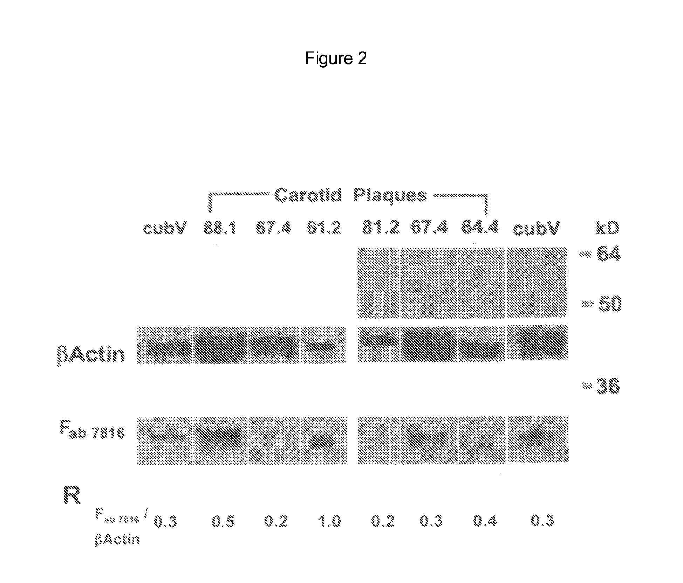

Sections used in WB (Figure 2) were taken from distinct area of the lesion. From the WBs the antigen is differentially distributed among patients and seems to be differentially expressed in distinct regions of the plaque

### Example 7. Identification of a bacterial antigen with Fab7816

SDS-PAGE was performed according to standard techniques. Briefly, protein concentration of all lysates was determined by BCA kit (Pierce) according to manufacturer instruction. SDS-PAGE loading buffer was added to protein lysates before heating at 90°C for 5 min. 8 µg of total protein lysate were loaded in each well and the gel was run for about 2 hours at 120V. PVDF membrane was treated as described in manufacturer instruction and proteins were transferred to membrane at 0,350 mA for 2 hours. The membrane was stained by Ponceau red to verify the proper transfer. Then the membrane was blocked with a solution of PBS1x/BSA10% at 4°C in agitation overnight. The day after the membrane was incubated with Fab E7816 (10 µg/ml) diluted in PBS1x/BSA5% for 1 hour at R.T. and then was washed three times with PBS1 x/Tween-20 0,1%. The membrane was then incubated for 1 hour at R.T. with anti-Human Light Chain K antibody conjugated with HRP and then washed three times with PBS1x/Tween-20 0,1%. The membrane was developed by SuperSignal West Pico Chemiluminescent Substrate (Pierce) according to manufacture instructions and the films were impressed for 1 and 5 min.

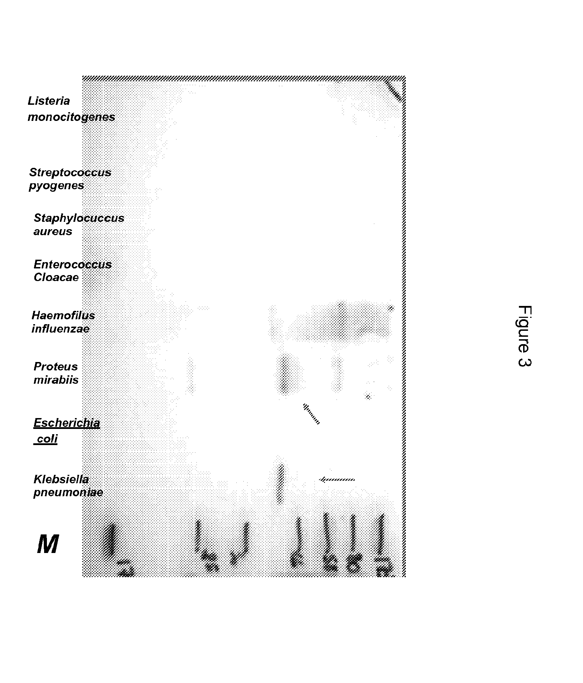

The gel shows that Fab7816 binds with high affinity and specificity to an antigen present in *Klebsiella Pneumoniae* and in *Proteus mirabilis* (Figure 3).

### Example 8: ompK36 from Klebsiella Pneumoniae

The sequence of a putative bacterial antigen OmpK36 was amplified from klebsiella lysate and cloned into a pET28b (Novagen) expression vector by using the following primer pairs containing two distinct 5' restriction endonuclease sequences: OmpK36NcolFW (SEQ ID NO:71) and OmpK36XholRW (SEQ ID NO:72); OmpK36BamHIFW (SEQ ID NO:73) and OmpK36XhoIRW (SEQ ID NO:72) and the following thermal profile was used: 95°C 5 min then 95°C 30min, 55°C 30min and 72°C 1 min. for 30 cycles; 72°C 10 min. The amplification products and the pET28b vectors were double digested for 4 hours with the following restriction endonucleases: NcoI/XhoI (New England Biolabs) or BamHI/XhoI (New England Biolabs), loaded onto a 1% agarose gel and purified by using a Qiagen gel extraction kit. The corresponding digested amplicone was overnight ligated by using 10 units of T4 ligase (Roche). After transformation of bacterial cells (XL1-Blue *E*. *coli)* and plating, 8 individual colonies for each ligation were inoculated on LB broth and grown overnight. After plasmid purification (Qiagen Miniprep kit) the inserts were sequenced with the following primers: T7FWpET (SEQ ID NO:74) and T7termpET (SEQ ID NO:75). Correct cloning was confirmed and one plasmid for each ligation was used for ompK36 expression. Nucleotide and amino acid sequences correspond to SEQIDNO: 76 and 77 respectively.

The BamH1 cloning allowed the generation of a 6xHISompK36 expression vector. Expression were carried out using BL21 (DE3) *E*. *coli* bacterial cells transformed with selected plasmids, than the protein was purified by Ni-NTA purification (QiaExpressionist - Qiagen).

Western Blot was performed according to the procedure described in example 7 on total bacterial protein extracts obtained as described above from IPTG induced or non-induced BL21 (DE3) cultures or on purified ompK36 protein, loading 1 µg of purified protein or 20 µl for total lysate. The membrane was incubated for 1 hour at R.T. with anti-Human Light Chain K antibody conjugated with HRP and then washed three times with PBS1x/Tween-20 0,1 %. The membrane was developed by SuperSignal West Pico Chemiluminescent Substrate (Pierce) according to manufacture instructions and the films were impressed for 1 and 5 min. WB was also developed with anti-HIS antibody (Roche) to verify the expression of HIS-tag at N-term of ompK36. Only the BamHI/XhoI cloned ompK36 has the HIS-tag as expected.

### Example 9: Expression and purification of IqG7816 by baculovirus in insect cells

Heavy and light chains of Fab 7816 were cloned in pAc-k-Fc vector (PROGEN cat N° PR3001) to allow the production of baculovirus particle. Baculovirus amplification and subsequent expression of IgG7816 in sf9 (Invitrogen) insect cells were performed following manufacturer instruction. The optimal MOI for IgG production and time for supernatant collection were evaluated. Sf9 cells were infected with a 0.2 MOI and grown for 5 days at 27°C in agitation. At day 5, cell culture was centrifuged at 2000RPM for 15 min. and the supernatant was harvested, filtered and PMSF (Sigma-Aldrich) was added (final 1 mM). The supernatant was loaded on a cromatographic column with protein G resin (Flow Rate: 0.8 ml/min for 18 h). The resin was washed with 100-150 ml of PBS 1x pH:7.4 (F.R.: 1-2 ml/min), and the bound IgG was then eluted with 10 ml of Citric Acid 0.1 M pH: 5.2 and the solution neutralized at pH: 7 with 1,2 ml of Tris 2M pH:11. The optimization of IgG production with different MOIs (multiplicity of infection) and time for supernatant collection was performed using the following ELISA assay. In brief, the day before, an ELISA plate was coated with 100 ng/well of Anti-Human Fab Antibody [Sigma-Aldrich] in 25 µl at 4°C O/N [or 2h at 37°C]. The day after every well was washed with ddH₂O and blocked with 180 µl/well of PBS/BSA 1% for 1 h at 37°C. The standard curve (from 250 ng/ml to 0.97 ng/ml, nine 1:2 serial dilutions of Standard IgGs(Sigma-Aldrich)) and at least three dilutions for every unknown samples (1:500, 1:1000 and 1:2000 at least in double) was added to the wells. In every well 40 µl of samples and standard were added and incubated for 1 h at 37°C. After 5 washes with PBS/Tween-20 0.1% an Anti-Human Fc antibody-HRP (SIGMA-Aldrich - diluted 1:8000), was added and incubated for 45-60 min. at 37°C .After five washes with PBS/Tween-20 0.1% 40 µl of TMB solution (Pierce) in every well was added and incubated for 15 min. at 37°C. After addition of 40 µl of Stop Solution (H₂SO₄ 0.1M) in every well the plates were read at 450 nm.

GraphPad Prism was used to draw the standard curve and interpolate the unknown sample quantities.

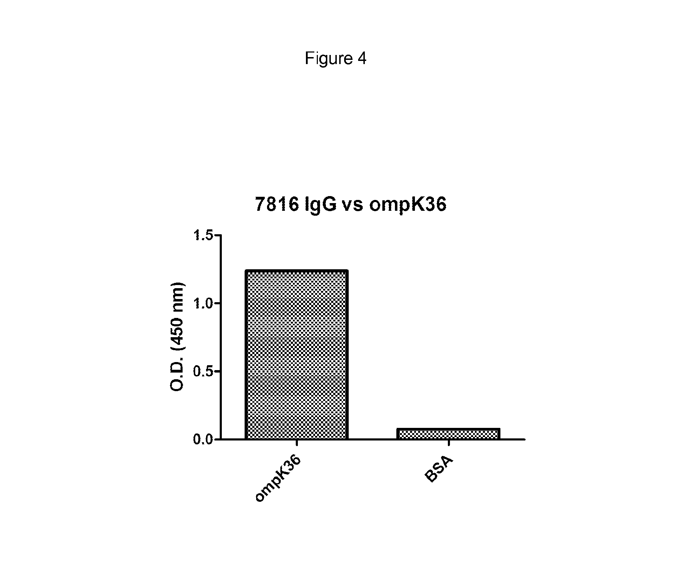

Eight micrograms of OmpK36 was coated on a 96 well ELISA plate in PBS, ph 5.0, overnight at 4°C. The day after, after three washes with PBS, the plate was blocked with PBS/BSA 1% , and purified 7816IgGs were added (9 µg/ml) to each well. After washing three timed with PBS/tween-20 0,1%, an anti-Human Fc antibody-HRP (SIGMA-Aldrich - diluted 1:8000), was added and incubated for 45-60 min. at 37°C. After five washes with PBS/Tween-20 0.1% 40 µL of TMB solution (Pierce) in every well was added and incubated for 15 min. at 37°C. After addition of 40 µl of Stop Solution (H₂SO₄ 0.1 M) in every well the plates were read at 450 nm. In figure 4 it is shown that 7816 when produced as a full IgG1 binds specifically to ompK36.

### Example 10: 2D electrophoresis Western Blotting (2DE-WB)

Sodium carbonate, glycine, sodium dodecyl sulfate (SDS) solution (20%) were purchased from Fluka. Ammonium persulfate, trizma base, urea, 3-[(3-Cholamidopropyl)dimethylammonio]-1-propanesulfonate (CHAPS), thiourea, bromophenol blue, iodoacetamide (IAA), N,N,N',N'-tetramethylethylenediamine (TEMED), 1,4-dithioerythritol (DTE), PBS, Tween -20, bovine serum albumin (BSA), cocktail of protease inhibitors and antibodies (mouse anti-FLAG antibody; anti-mouse-peroxidase conjugated antibody) were obtained from Sigma. Agarose, glycerol (87% w/w), DryStrip Cover Fluid, DeStreak reagent, IPG (Immobilised pH Gradient) buffer pH 3-10NL, Immobiline DryStrip pH 3-10 NL, 7 cm, MW protein standards, PVDF membrane and ECL plus Chemiluminescent Substrate were from GE Healthcare. Coomassie brilliant blue G-250, 30% Acrylamide/Bis solution 37.5:1 and Bradford Protein Assay were purchased from Bio-Rad. Acetic acid and methanol were purchased from Merck. Zwittergent was from Calbiochem.

A piece of the coronary plaque was weighted (65 mg), cut in small fragments and directly smashed with a Dounce in 500 µl of the R5 buffer, containing: urea 8M, thiourea 2M, CHAPS 4%, Zwittergent 0.05%, 40mM trizma base and a cocktail of protease inhibitors. The lysate was then sonicated for 5 minutes and centrifuged at 13,000 rpm for 30 minutes at 15°C. Supernatant protein content was evaluated by Bradford assay, using BSA dissolved in the same buffer, as standard. The proteins (200 µg), dissolved in R5 buffer (final volume 130 µl), were added to DeStreak (100 mM) and 2% IPG buffer pH 3-10NL, prior to loading the sample on strip pH 3-10NL, 7 cm. Total focusing run for the 1^{st} dimension was 50,000 Vh. The 2^{nd} dimension was carried out using 12.5% acrylamide SDS-PAGE. MW Standard proteins were run on the same gel. The gel was stained with colloidal Coomassie Brilliant Blue (cCBB).

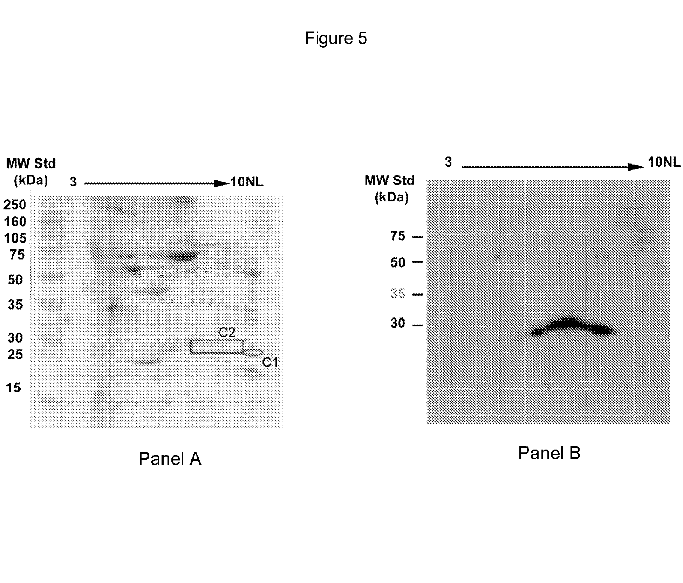

Similarly, 70 µg proteins dissolved in R5 buffer (final volume 130 µl), were added DeStreak (100 mM) and 2% IPG buffer pH 3-10NL, prior to loading the sample on strip pH 3-10NL, 7 cm for the gel to be blotted. Total focusing run for the 1^{st} dimension was 50,000 Vh. The 2^{nd} dimension was carried out using 12.5% acrylamide SDS-PAGE. MW Standard proteins were run on the same gel. Proteins were then transferred to PVDF membrane by semi-dry electroblotting and probed with primary antibody 7816FLAG. Immuno-complexes were visualized by incubation with anti-FLAG, peroxidase-conjugated secondary antibody and chemiluminescent detection (see figure 5, panel A and B).

### Example 11: Protein identification by MALDI-ToF mass spectrometry

Protein corresponding to positive spots in WB were excised from the gel, destained, reduced with DTE (10 mM in ammonium bicarbonate 25mM) at 56°C for 30 minutes, alkylated with IAA (55mM in ammonium bicarbonate 25mM) in the dark at room temperature for 20 minutes. They were digested with trypsin and directly analysed by MALDI-ToF mass spectrometry, using alpha-cyano-4-hydroxycinnamic acid as matrix. We utilized Mascot software (Matrix Science, London) for protein searching in IPI_human-20100623 database and the standard parameters (tryptic digest with a maximum of two missed cleavages, carboxyamidomethylation of cysteine residues, partial methionine oxidation and a mass tolerance of 50 ppm). We accepted an identification when the Mascot score was >66, with a good sequence coverage.

By comparison of images of 2DE and 2DE-WB described in example 10, it was decided to analyse the spots indicated in Figure 5, panels A and B. They were recognized by the primary antibody we used. By mass spectrometry analysis transgelin 1 (SM22) was unequivocally identified as the putative natural (self) antigen recognized by Fab 7816 after sampling two distinct spots in the 2D gel. Data have been confirmed on purified protein by protein sequencing analysis.

### Example 12: Identification of Fab 5LcX

Biopanning of library 11 (ID11 LIB) was carried out again on recombinant ompK36 coated on ELISA plates, for three rounds of selection.

Twenty clones were sequenced: the majority of clones resulted to have 7816 heavy and/or light sequences.

One clone, named Fab 5LcX, carried a different combination of light sequence SEQ ID NO 5 coding for the amino acidic sequence corresponding to SEQ ID NO: 6, and heavy sequence SEQ ID NO 7 coding for the amino acidic sequence corresponding to SEQ ID NO: 8.

### Example 13: Identification of Fab 1630

The phage display Fab library from plaque 12 (ID12LIB) was screened once more by biopanning on the bacterial lysate and selected phages were tested on purified ompk36 in ELISA by biopanning carried out as described in example 2.

Seven selected clones were further sequenced, among them the most relevant was Fab1630 consisting of light chain SEQ ID NO 9 coding for the amino acidic sequence corresponding to SEQ ID NO: 10, and heavy chain SEQ ID NO 11 coding for the amino acidic sequence corresponding to SEQ ID NO: 12.

ELISA against purified ompK36 carried out with a phage library made from peripheral blood sample of patient ID12 allowed to further identify the following heavy and light chains in the Sequence Listing: HC SEQ ID NO from 17 to 27 (odd numbers), coding for the aa SEQ ID NO from 18 to 28 (even numbers) and LC SEQ ID NO from 29 to 33 (odd numbers), coding for the aa SEQ ID NO from 30 to 34 (even numbers).

### Example 14: Western blotting on commercial transgelin 1 with Fab7816

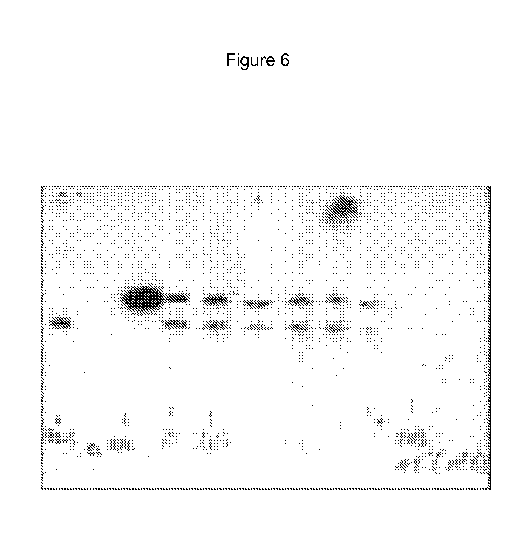

SDS-PAGE was performed according to what described in example 7. SDS-PAGE loading buffer was added to the recombinant transgelin (rTAGLN - Origene cod: TP302448) and was heated at 95°C for 5 min then 400 ng of rTAGLN were loaded in each well and the gel was run for about 2 hours at 120V. PVDF membrane were treated as indicated in the manufacturer's instruction and proteins were transferred to membrane at 0.350 mA for 2,5 hours. The membrane was stained by Ponceau red to verify the transfer. The membrane was then blocked with a solution of PBS1x/BSA10% at 4°C in agitation overnight. The day after the membrane was incubated with Fab E7816Flag (Lot R004/10 - 11/05/2010, 10 µg/ml) or IgG7816 (4 µg/ml) diluted in PBS1x/BSA5% for 1 hour at r.t. and then washed 3 times with PBS1x/Tween-20 0,1 %. The membrane was incubated for 1 hour at r.t. with anti-Human Light Chain K antibody conjugated with HRP (Sigma cod: A7164) and then washed two times with PBS1x/Tween-20 0,1% and one time with PBS1x. Two wells were incubated with control antibody: anti-MYC-tag conjugated with HRP (Abcam cod: ab62928) and anti-TAGLN (Abnova cod: H00006876-M01). The anti-TAGLN was revealed by anti-Mouse-Fab conjugated with HRP (SIGMA cod: A9917). The membrane was developed by SuperSignal West Pico Chemiluminescent Substrate (Pierce) according to manufacture instructions and the films were impressed for 1 and 3 min. In the last lane a human monoclonal Fab against Influenza (PN-SIAFab49) was used as a negative control. The autoradiographic film is shown in Figure 6.

### Example 15: Western blotting on commercial transgelin with Fab1630

The binding of Fab1630 to transgelin was evaluated by Western Blot as described in the above example 14 by using recombinant transgelin, Myc- and Flag-tagged. Transgelin was run in SDS-PAGE and one lane was stained with Coomassie blue to indentify the correct band size. Transgelin shows two bands at different molecular weight (27 kDa and 22 kDa) possibly due to post-translational modifications.

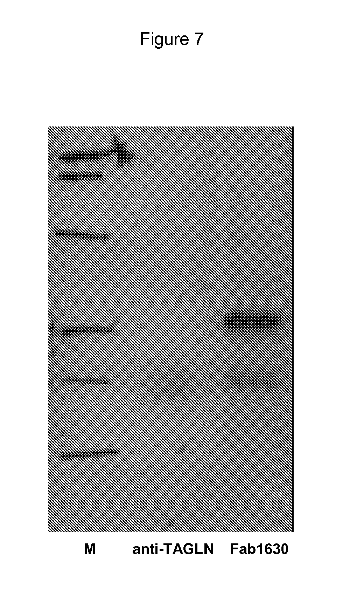

The assay demonstrates that Fab1630 binds specifically to transgelin (both bands), anti-Flag antibody binds to both bands too, anti-Myc antibody bind only to the heavier band and anti-TAGLN antibody binds only to the lighter band (see figure 7).

## Claims

1. An antibody immunologically reacting with human transgelin or fragments thereof and with a protein with at least 50% similarity to OmpK36 (Outer membrane protein, Klebsiella, K36; GI: 295881594) or fragments thereof.

2. The antibody according to claim 1 wherein said transgelin is transgelin 1 (Accession N° Q01995, GI:48255907).

3. The antibody according to claim 1 comprising a set of CDRs 1-3 selected in the group consisting of: set 1) SEQIDNO: 36, 38 and 40; set 2): SEQIDNO: 54, SEQIDNO: 56 SEQIDNO: 58, for a Heavy chain, and to set 3) SEQIDNO: 42 and 44 and set 4) to SEQIDNO: 60 and 62, for a light chain.

4. The antibody according to any one of claims 1-3 comprising at least one of the following Heavy chains selected in the group consisting of: SEQIDNO:2, SEQIDNO:6, SEQIDNO:10, and at least one of the light chains selected in the group consisting of SEQIDN0:4, SEQIDNO:8, SEQIDNO:12.

5. An antibody which is human and recombinant, obtainable by expression of the nucleotidic sequences encoding a heavy chain selected from the group consisting of: SEQIDNO:2, SEQIDNO:6, SEQIDNO:10 and a light chain selected from the group consisting of: SEQIDNO:4, SEQIDNO:8, SEQIDNO:12.

6. The antibody according to any one of claims 1-5 wherein the Heavy chain comprises or consists of SEQIDNO:2 and the Light chain comprises or consists of: SEQIDNO:4.

7. The antibody according to any one of claims 1-5 wherein the Heavy chain comprises or consists of SEQIDNO:10 and the Light chain comprises or consists of:SEQ!DNO:12.

8. The antibody according to any one of claims 1-7 further recognizing an antigen in the atherosclerotic plaque.

9. The antibody according to any one of claims 1-8 for the preparation of an immunodiagnostic reagent for the detection of an atherogenic process or an atheromatous disease.

10. A method for detecting antibodies against an antigen in an atherosclerotic plaque comprising allowing an unknown biological sample to react with human transgelin or fragments thereof, optionally in competition with an antibody according to claims 1-9.

11. The method of claim 10 wherein the antibody comprises the following heavy and light chain combinations: SEQIDNO: 2 and SEQIDNO: 4, SEQIDNO: 6 and SEQIDNO: 8, SEQIDNO: 10 and SEQIDNO: 12, SEQIDNO: 14 and SEQIDNO: 16.

12. The method according to any one of claims 10-11 further comprising allowing said unknown biological sample to react with a protein homologous to OmpK36 or fragments thereof.

13. The method according to any one of claims 10-12 which is an immunoassay.

14. The method according to claim 13 selected from: Western-blot and ELISA assay.

15. The method according to any one of claims 10-14 for the detection of an atherogenic process or an atheromatous disease.

16. A method for detecting an antigen in an atherosclerotic plaque of an unknown sample comprising allowing said unknown biological sample to react with an antibody comprising at least a heavy chain selected from: SEQIDNO: 2, 6, 10, 14 and at least a light chain selected from the group consisting of: SEQIDNO: 4, 8, 12, 16.

17. Transgelin 1 as a marker of the presence of an atherosclerotic plaque or of atherogenesis.

18. Use of Transgelin 1 as a marker of the presence of an atherosclerotic plaque, an atherogenic process or atheromatous disease.

19. The use according to claim 18 in an immunoassay in combination with a bacterial outer membrane protein selected among those with at least 50% similarity to OmpK36.

20. Use of an antibody according to any one of claims 1-9 for the diagnosis of an atherogenic process or an atheromatous disease.

21. The use according to claim 20 wherein said atheromatous disease is selected in the group consisting of:
- atherogenic ischemic or occlusive evolution in an arterial vessel,
- Acute Coronary Syndrome comprising: unstable angina, ST Elevation Myocardial Infarction (STEMI), non STEMI myocardial infarction and related cardiovascular diseases,
- intra-cerebral occlusive diseases
- peripheral artery occlusive diseases
- non acute coronary diseases.
